(19) Europäisches Patentamt
European Patent Office
Office européen des brevets

(11) **EP 4 538 706 A1**

(12) **EUROPEAN PATENT APPLICATION**

(43) Date of publication:
**16.04.2025 Bulletin 2025/16**

(21) Application number: **24206734.6**

(22) Date of filing: **15.10.2024**

(51) International Patent Classification (IPC):
***G01N 33/574*** *(2006.01)*

(52) Cooperative Patent Classification (CPC):
**G01N 33/57407; G01N 33/57488;** G01N 2333/521;
G01N 2400/38

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **15.10.2023 PL 44638923**

(71) Applicant: **Warszawski Uniwersytet Medyczny**
**02-091 Warszawa (PL)**

(72) Inventors:
• **LACHOTA, Mieszko**
**01-199 Warszawa (PL)**

• **ZIELNIOK, Katarzyna**
**01-912 Warszawa (PL)**
• **ZAGOZDZON, Radoslaw**
**05-080 Truskaw (PL)**
• **GOZDZ, Agata**
**02-103 Warszawa (PL)**
• **SZPAK, Patrycja**
**01-307 Warszawa (PL)**
• **KALASZCZYNSKA, Ilona**
**02-795 Warszawa (PL)**
• **CZERNICKI, Tomasz**
**05-816 Opacz-Kolonia (PL)**

(74) Representative: **Patpol Kancelaria Patentowa Sp. z o.o.**
**Nowoursynowska 162J**
**02-776 Warszawa (PL)**

(54) **NEW MARKERS FOR DIAGNOSING GLIOBLASTOMA MULTIFORME AND USES THEREOF**

(57) The invention relates to methods of diagnosing glioblastoma multiforme in a subject, monitoring the subject's response to glioblastoma multiforme treatment, and monitoring the progression or recurrence of glioblastoma multiforme based on the use of a combination of the following five markers: the chemokine CCL22 (CCL22), glial fibrillary acidic protein (GFAP), midkine (MDK), neurogranin (NRGN), and osteopontin (SPP1). The invention also relates to uses of combinations of the aforementioned markers: CCL22, GFAP, MDK, NRGN, and SPP1 for diagnosing or monitoring glioblastoma multiforme.

EP 4 538 706 A1

**Description**

The invention relates to methods of diagnosing glioblastoma multiforme in a subject, monitoring the subject's response to treatment for glioblastoma multiforme, and monitoring the progression or recurrence of glioblastoma multiforme based on the use of a combination of the following five markers: the chemokine CCL22 (CCL22), glial fibrillary acidic protein (GFAP), midkine (MDK), neurogranin (NRGN), and osteopontin (SPP1). The invention also relates to uses of combinations of the above-indicated markers: CCL22, GFAP, MDK, NRGN, and SPP1.

Glioblastoma multiforme (GBM) is the most common malignant tumor in adults among the diverse group of tumors that constitute primary brain tumors (i.e. those that originate from cells of the central nervous system). Glioblastoma multiforme is most often diagnosed in people between 55 and 65 years of age, but patients include both people under 20 and those over 65 years of age. The majority of cases are GBM arising *de novo,* which mainly affects elderly people (90% of diagnoses), however, glioblastoma multiforme can develop secondarily from previously existing lower-grade gliomas, which is more common in younger patients (10% of cases) [Vitovcova, B., et al., Biology of Glioblastoma Multiforme-Exploration of Mitotic Catastrophe as a Potential Treatment Modality. Int J Mol Sci, 2020. 21(15)]. Regardless of the age and disease variant, the prognosis for all patients with glioblastoma multiforme is poor, and their survival is usually little more than two years from diagnosis (an average of 12-15 months [Pearson, J.R.D. and T. Regad, Targeting cellular pathways in glioblastoma multiforme. Signal Transduct Target Ther, 2017. 2: p. 17040]). Apart from a small percentage of patients who develop this tumor as a result of having rare genetic syndromes, such as neurofibromatosis, Li-Fraumeni syndrome or Lynch syndrome, the cause of glioblastoma multiforme most often remains undetermined, and the only established risk factor is exposure to ionizing radiation (occurring, for example, during therapeutic radiotherapy of eye and brain tumors in children, or leukemia). Like in case of other tumors, the key issue for the patient is to diagnose the tumor at the earliest possible stage, but early diagnosis is complicated by the fact that the symptoms of the disease are often general and nonspecific, or completely absent. Patients most often report to a doctor complaining of neurological symptoms that have been progressing for several days/weeks, which most often include: headache (in 50-60% of patients), seizures (20-50%), and, depending on the location of the tumor, focal neurological symptoms, such as cognitive and personality changes, language deficits, muscle weakness, memory loss, or visual symptoms (10-40%) [Chang, S.M., et al., Patterns of care for adults with newly diagnosed malignant glioma. JAMA, 2005. 293(5): p. 557-64.] Diagnostic procedure in the case of suspected brain tumor has been based for many years on imaging diagnostics (magnetic resonance imaging of the brain with contrast), however, to make the diagnosis of glioblastoma multiforme, histopathological and molecular evaluation of the neoplastic tissue is necessary, which is possible after its resection (most often) or, less frequently, stereotactic biopsy (mainly in the case of unresectable changes or those located in a critical location). This means that a patient with non-specific and low-intensity symptoms first waits several months for an imaging test, then for its description and scheduling a date for a surgery, and only after the brain tumor removal surgery and another two or three weeks of waiting for the result of the histopathological tests the diagnosis is made. The time of diagnosis and implementation of treatment is the only modifiable factor influencing the life expectancy of a patient with a particularly rapidly developing tumor, such as glioblastoma multiforme. It is a factor that often deprives the patient of treatment options, and even in properly diagnosed patients who underwent radio- and chemotherapy after resection of the tumor, the tumor recurs in most cases. And again, the only method of monitoring the effects of the treatment and diagnosing the recurrence is the magnetic resonance imaging with contrast.

Attempts have been made to define single markers as diagnostic markers associated with the development of glioblastoma multiforme. For example, Jones, D.R. in "Measuring midkine: the utility of midkine as a biomarker in cancer and other diseases." Br J Pharmacol, 2014. 171(12): p. 2925-39, describes the usefulness of midkine level determination as a biomarker of cancer and other diseases. However, since its level is increased in many pathological conditions, midkine is not a specific biomarker for glioma. Therefore, midkine, as well as other plasma proteins, as a single marker, are not a desirable target for diagnostic methods.

An attempt to identify proteins as potential blood-based biomarkers for malignant glioma was also disclosed in the publication by Lange, R.P., et al. (Evaluation of eight plasma proteins as candidate blood biomarkers for malignant gliomas. Cancer Invest, 2014. 32(8): p. 423-9). Eight brain-derived proteins (GFAP, NRGN, BDNF, ICAM-5, MT-3, SNCB, and S100B) were evaluated, however, as compared to healthy controls, none of the proteins appeared to be specific for glioblastoma multiforme.

Invasive methods are also known for identification of the set of genes involved in the development of GBM, by assessing the expression profiles of GBM tissues obtained from patients using cDNA microarrays (see, e.g., Yokota, T., et al., Identification of histological markers for malignant glioma by genome-wide expression analysis: dynein, alpha -PIX and sorcin. Acta Neuropathol, 2006. 111(1): p. 29-38). Comparison of GBM profiles with those of normal brain tissue identified a number of differentially expressed genes: 54 overexpressed and 45 underexpressed genes in GBM, and indicated overexpression of DNCH2, ARHGEF6, NPM1 and SRI and underexpression of NRGN and TM4SF2. Immunohistochemical staining of the three relevant gene products, dynein (a DNCH2 product), alpha-PIX (an ARHGEF6 product) and sorcin (a SRI product) indicated that this technique may be useful for the histological assessment of glial tumors.

Additionally, noninvasive methods for identifying markers for use in diagnosing glioblastoma multiforme, monitoring tumor progression, and detecting tumor recurrence after treatment, are known in the art. For example, the U.S. patent document US20140045915A1 discloses methods for assaying a biological sample from a subject, such as a blood, serum, plasma, or urine sample, by analyzing components of microvesicle fractions in determining of a risk, diagnosis, prognosis, monitoring of or directing a treatment. One of the neoplastic diseases identified therein is glioblastoma. Also disclosed are methods for treating and identifying biomarkers using microvesicle fractions, as well as kits, pharmaceutical compositions, and profiles related to these methods. The specification provides an extensive list of genes with somatic mutations associated with cancer, including CCL22, GFAP, MDK, NRGN, and SPP1.

Similarly, the US patent application publication US20220244263A1 provides a method comprising measuring the expression level of one or more biomarkers in a biological sample from a patient suffering from a tumor, including glioblastoma multiforme. The list of possible markers to be used includes hundreds of markers, including, among others, CCL22, GFAP, MDK, NRGN and SPP1, the gist of the invention being to determine the absolute signature weight, the absolute value of which for at least two of the five determined biomarkers is greater than 0.025.

However, the presented marker sets do not allow for unequivocal, non-resectable and screening diagnosis of glioblastoma multiforme in a blood, plasma or serum sample. Thus, the state of the art lacks diagnostic tests that could quickly and in a low-invasive way indicate patients with a high probability of glioblastoma multiforme presence, who should be diagnosed and treated urgently. Additionally, the state of the art does not provide markers specific only for GBM, which would indicate the presence of this tumor in a sufficiently specific and sensitive manner.

The inventors unexpectedly identified a combination of biological markers present in blood plasma - CCL22, GFAP, MDK, NRGN and SPP1, the simultaneous assessment of which in a plasma sample can be used to screen patients with suspected primary GBM or its recurrence, monitor GBM progression, or a response to treatment.

Thus, in the first aspect, the invention provides a method of diagnosing glioblastoma multiforme (GBM) in a subject, comprising

(a) a step of determining concentrations of a combination of the following five markers: CCL22, GFAP, MDK, NRGN and SPP1, in a sample collected from the said subject, and
(b) a step of determining a risk factor using the concentrations determined in step (a), comparing it with a predetermined cut-off point and determining on that basis the presence or absence of glioblastoma multiforme in the subject.

In the second aspect, the invention provides a method of monitoring a response of a subject to GBM treatment, comprising

(a) a step of determining concentrations of a combination of the following five markers: CCL22, GFAP, MDK, NRGN and SPP1, in the sample collected from the subject at the first time point;
(b) a step of determining concentrations of the above-mentioned combination of markers in a sample collected from the subject at a second time point;
(c) a step of determining risk factors using the concentrations determined in steps (a) and (b), comparing them with the predetermined cut-off point and, on this basis, determining whether the subject is a responder to treatment for glioblastoma multiforme.

Preferably, in the method of monitoring the response to GBM treatment according to the invention, steps (a), (b) and (c) are repeated. Thus, during or after treatment of the subject, it is possible to obtain information as to whether the treatment has produced the expected effect.

In a third aspect, the invention provides a method of monitoring the progression or recurrence of GBM in a subject, comprising

(a) a step of determining concentrations of a combination of the following five markers: CCL22, GFAP, MDK, NRGN and SPP1, in the sample collected from the subject at the first time point;
(b) the step of determining concentrations of the above-mentioned combination of markers in a sample collected from the subject at a second time point;
(c) a step of determining risk factors using the concentrations determined in steps (a) and (b), comparing them with the predetermined cut-off point and, on this basis, determining the progression or recurrence of glioblastoma multiforme.

Preferably, in the method of monitoring the progression or recurrence of GBM according to the invention, steps (a), (b) and (c) are repeated. Thus, it is possible to assess the condition of a subject suffering from GBM or to monitor a subject who is at increased risk of developing GBM (e.g., a subject previously diagnosed with GBM or a lower grade glioma).

In all of the above methods of the invention, the cut-off point is determined by analyzing the concentrations of the markers CCL22, GFAP, MDK, NRGN and SPP1 determined in samples collected from healthy subjects, samples collected from subjects with glioblastoma multiforme, and/or samples collected from subjects with another type of tumor. Based on the analysis of the determined values of CCL22, GFAP, MDK, NRGN and SPP1 marker concentrations, a risk factor value is determined, which, if lower than the cut-off point value, indicates that the patient is healthy, and those for which the calculated risk factor "Y" is equal to or higher than the cut-off point value, are classified as samples originating from/collected from subjects with glioblastoma multiforme.

In a preferred embodiment of the invention, the risk factor "Y" is defined by the following formula:

$$Y = Midkine \left[\frac{pg}{ml}\right] * 2.311382e^{-05} + Osteopontin \left[\frac{pg}{ml}\right] * 5.020056e^{-06} + Neurogranin \left[\frac{pg}{ml}\right] * 7.851757e^{-04} + CCL22 \left[\frac{pg}{ml}\right]$$

$$* -5.500833e^{-04} + GFAP \left[\frac{pg}{ml}\right] * -3.058356e^{-04}$$

In the above equation, the risk factor Y is calculated using multivariate linear regression - and it is the sum of the concentrations of five markers CCL22, GFAP, MDK, NRGN and SPP1, where the concentration of each one is multiplied by the individual regression coefficient. The cut-off point for the risk coefficient "Y" amounts to 0.2472802. It means that all samples for which the risk factor "Y" calculated based on the determined concentration values of the markers CCL22, GFAP, MDK, NRGN and SPP1 is less than the cut-off point are classified as samples derived/collected from healthy subjects, and those for which the calculated risk coefficient "Y" is equal to or greater than the cut-off point are classified as samples derived/collected from subjects with glioblastoma multiforme. The essence of the formula are the values of the regression coefficients, which are positive (greater than zero) for MDK, NRGN and SPP1, and negative (less than zero) for CCL22 and GFAP.

$$Y = Midkine \left[\frac{pg}{ml}\right] * x1 + Osteopontin \left[\frac{pg}{ml}\right] * x2 + Neurogranin \left[\frac{pg}{ml}\right] * x3 + CCL22 \left[\frac{pg}{ml}\right] * x4 + GFAP \left[\frac{pg}{ml}\right] * x5$$

wherein:

$X^1 > 0;\ X^2 > 0;\ X^3 > 0;\ X^4 < 0;\ X^5 < 0$

This illustrates trends in marker concentration changes indicating a higher probability of diagnosing glioblastoma multiforme in patients with higher levels of MDK, NRGN and SPP1, and a lower probability in patients with elevated levels of CCL22 and GFAP. The optimal values of regression coefficients may change when attempting to refine the formula in a different patient population.

In a further aspect, the invention provides uses of the CCL22, GFAP, MDK, NRGN and SPP1 markers. Thus, the invention relates to the use of a combination of the CCL22, GFAP, MDK, NRGN and SPP1 markers for diagnosing glioblastoma multiforme by determining the concentration of the above-indicated markers in a sample collected from a subject.

The invention relates also the use of a combination of the CCL22, GFAP, MDK, NRGN and SPP1 markers for monitoring the response of a subject to treatment for glioblastoma multiforme by determining the concentration of the aforementioned markers in a sample collected from said subject.

Furthermore, the invention relates to the use of a combination of the CCL22, GFAP, MDK, NRGN and SPP1 markers for monitoring the progression or recurrence of glioblastoma multiforme by determining the concentration of the above-mentioned markers in a sample collected from a subject.

In all of the above methods and uses of the invention, the sample in which the concentrations of the CCL22, GFAP, MDK, NRGN and SPP1 markers are determined is preferably a whole blood, plasma or serum sample, more preferably a plasma sample. The above-mentioned markers may also be determined in other samples, such as the cerebrospinal fluid.

Preferably, the concentrations of the CCL22, GFAP, MDK, NRGN and SPP1 markers in the tested samples are determined using the Luminex method. However, it is possible to use other methods to determine the concentrations of markers in the sample, such as ELISA, mass spectrometry or flow cytometry.

All technical and scientific terms and definitions used herein have the same meaning as commonly understood by the one of skill in the art to which this invention pertains. The term "subject" refers to a human or non-human animal and may mean a patient under medical care. This includes patients who are at risk of developing GBM (e.g., patients previously diagnosed with GBM or lower grade glioma, or patients exposed to ionizing radiation), patients diagnosed with GBM, as well as subjects previously diagnosed with GBM. In a preferred embodiment of the methods of the invention, the subject is a human.

The terms "marker" and "biomarker" are used interchangeably herein and refer to a marker whose presence or a specific amount in a sample collected from a subject, when analyzed alone or in combination with other markers, allows for determination of the likelihood of a particular course or outcome. For example, when one or more markers or biomarkers reach a certain level in samples collected from patients, that level may signal that the subject has an increased likelihood of developing a particular disease, e.g., GBM, compared to subjects whose levels of the same markers or biomarkers are different. In accordance with the invention, markers are proteins whose concentration in the blood is affected by the presence or absence of GBM.

The term "diagnosing" refers to any method by which a person skilled in the art can assess and/or determine the presence or absence of a particular disease or condition (such as GBM) in a subject. The term does not imply that it is possible to determine the presence or absence of a particular disease with 100% accuracy. Instead, a person skilled in the art will understand that diagnosing refers to an increased likelihood that a subject has a particular disease. Diagnosing may be accomplished by analyzing concentrations of specific markers or biomarkers in a sample from a subject. In accordance with the invention, diagnosing GBM is accomplished by determining the concentration of a combination of the markers CCL22, GFAP, MDK, NRGN, and SPP1 in a sample collected from a subject and relating this concentration to the concentrations of the corresponding markers in healthy subjects, i.e., those who do not have GBM.

The term "monitoring" refers to the cyclical assessment of the patient's condition, e.g. by determining specific markers or biomarkers. Monitoring refers to determining the course of the disease, the effectiveness of treatment and the occurrence of disease relapses. It is determined during monitoring whether the level of markers of the subject changes towards the level found in the healthy population, which means the improvement of the subject's condition, or towards the level found in diseased people, which means the deterioration of the subject's condition or recurrence of the disease.

The technical solutions according to the invention developed by the inventors can be used in three aspects of the care of patients with glioblastoma multiforme. First, they can serve as an inexpensive, easily accessible and safe method of screening patients with family history or genetic burden increasing the risk of glioblastoma multiforme. Second, they can be used as a method of observing and monitoring patients after tumor resection for tumor recurrence. Due to their low cost, easy accessibility and low invasiveness, the methods and uses according to the invention enable more frequent monitoring of patients after tumor resection. Moreover, the results presented in the examples regarding the level of gene expression indicate that the combination of the five biomarkers according to the invention can serve as a non-invasive, "liquid" biopsy, enabling the differentiation of glioblastoma multiforme from other tumors of the central nervous system.

The results obtained by the Inventors will confirm the high diagnostic value of the combination of markers according to the invention compared to other types of cancers and other diseases. Therefore, this combination enables a quick and cheap method for an initial triage, monitoring of patients with suspected glioblastoma multiforme or its recurrence. The use of the combination of CCL22, GFAP, MDK, NRGN and SPP1 markers significantly increases the precision of the assessment of the risk of GBM occurrence.

The above and other advantages and benefits of the present invention will become more apparent from the following examples of embodiments of the invention.

The subject-matter of the invention is shown in the drawing, in which:

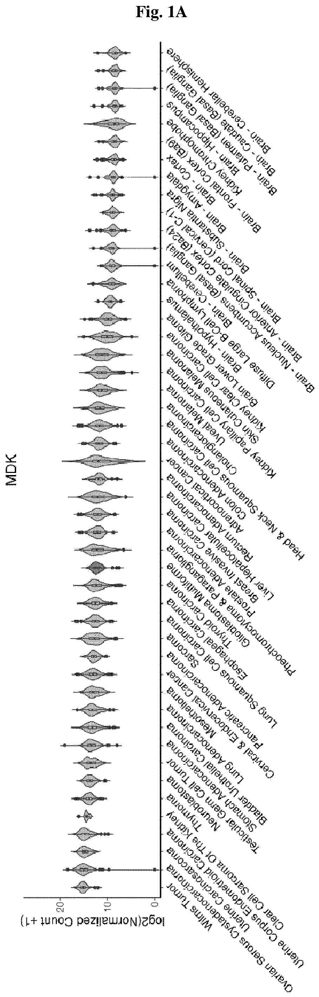

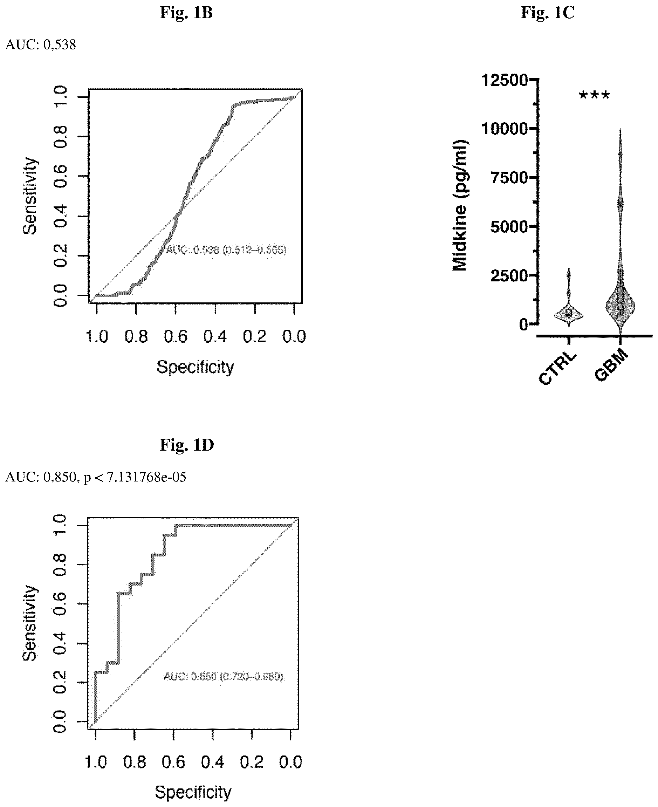

Figure 1 presents the expression level of midkine (MDK) A) at the gene level (in tissues) and C) at the protein level (in blood plasma) along with the receiver operating characteristic (ROC) curve analysis of the diagnostic value of a midkine assay as a marker at the level of B) genes among healthy brain tissues and other types of tumors and D) proteins in plasma compared to the control group of healthy subjects.

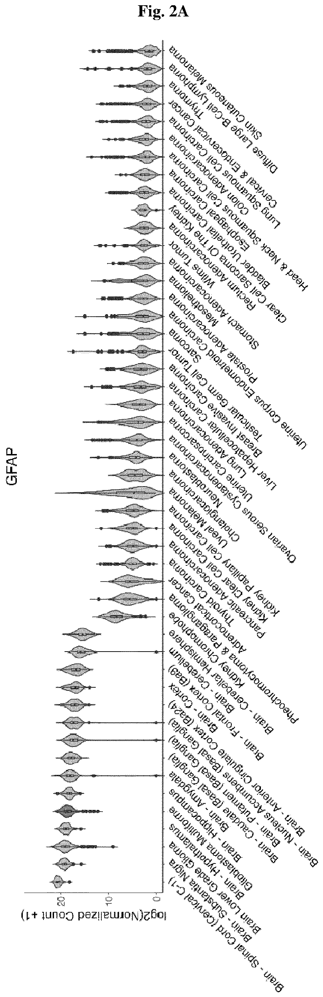

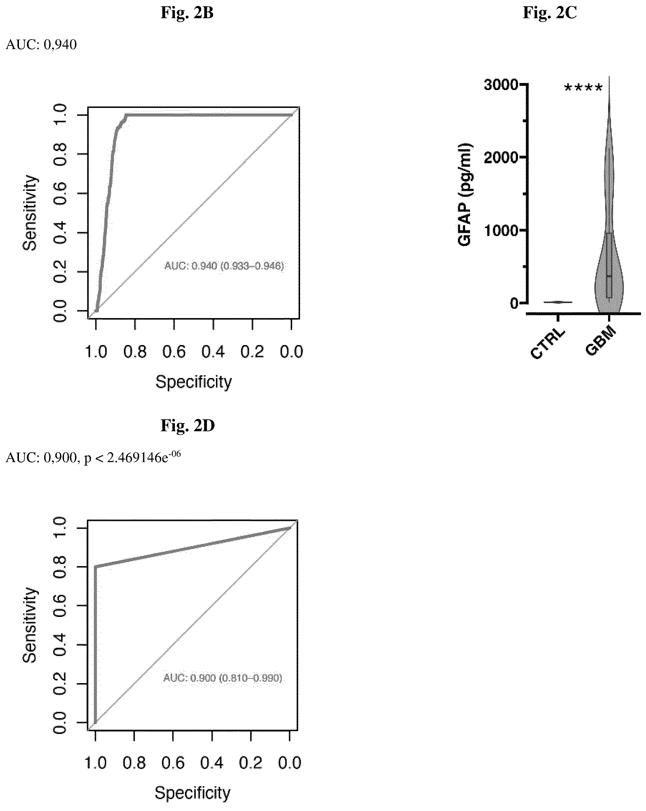

Figure 2 presents the expression level of GFAP A) at the gene level (in tissues) and C) protein level (in blood plasma) along with the ROC analysis of the diagnostic value of a GFAP assay as a marker at the level of B) genes among healthy brain tissues and other types of tumors and D) proteins in plasma compared to the control group of healthy subjects.

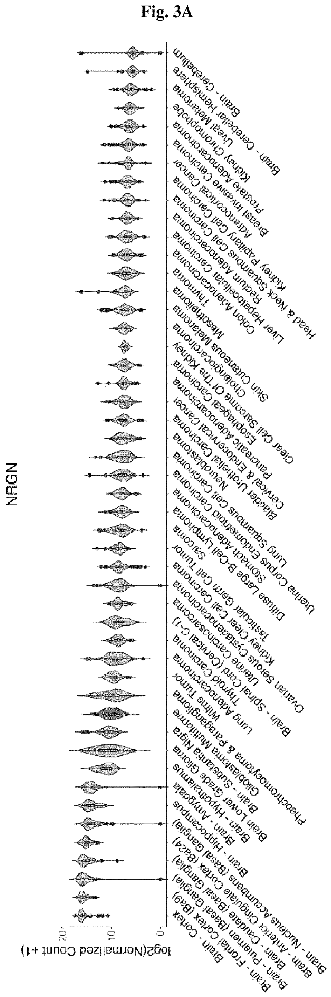

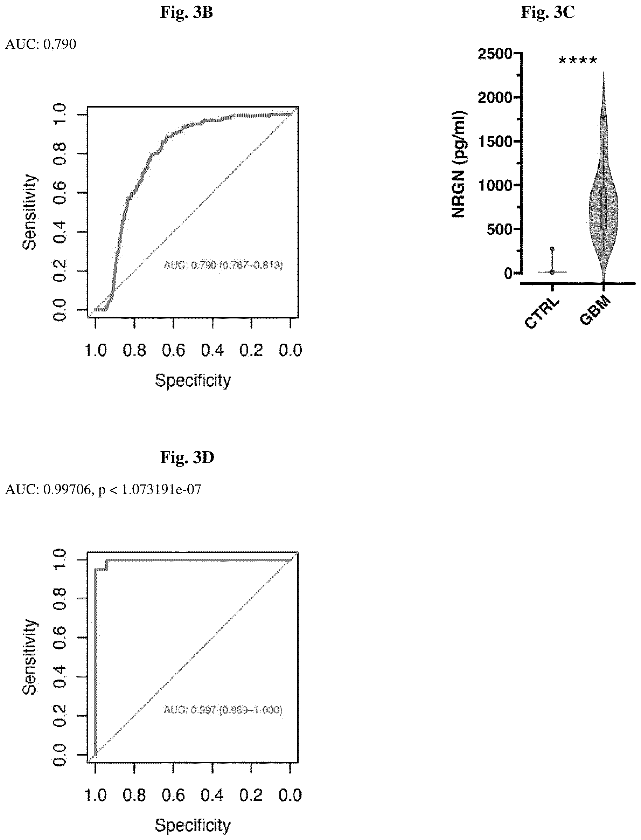

Figure 3 presents the expression level of NRGN A) at the gene level (in tissues) and C) protein level (in blood plasma) along with the ROC analysis of the diagnostic value of a NRGN assay as a marker at the level of B) genes among healthy brain tissues and other types of tumors and D) proteins in plasma compared to the control group of healthy subjects.

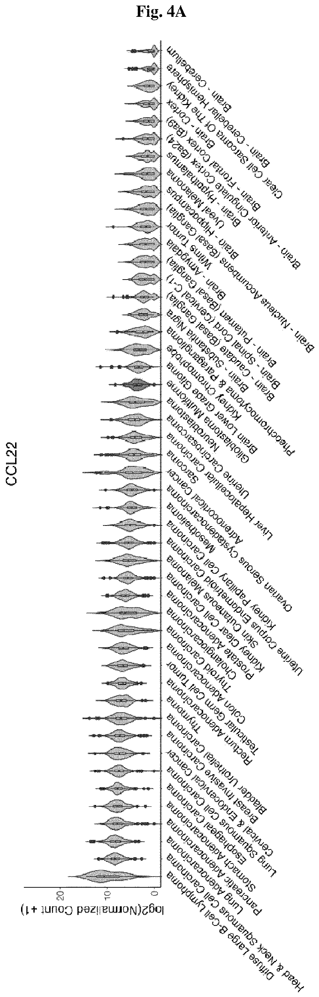

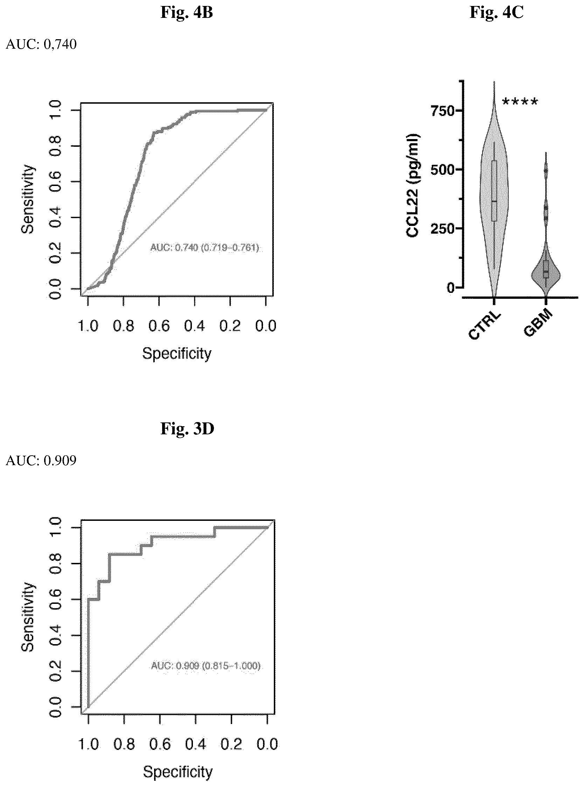

Figure 4 presents the expression level of CCL22 A) at the gene level (in tissues) and C) protein level (in blood plasma) along with the ROC analysis of the diagnostic value of a CCL22 assay as a marker at the level of B) genes among healthy brain tissues and other types of tumors and D) proteins in plasma compared to the control group of healthy subjects.

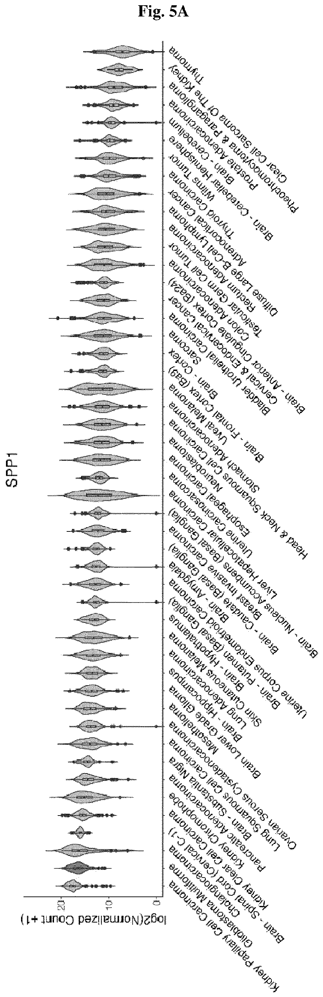

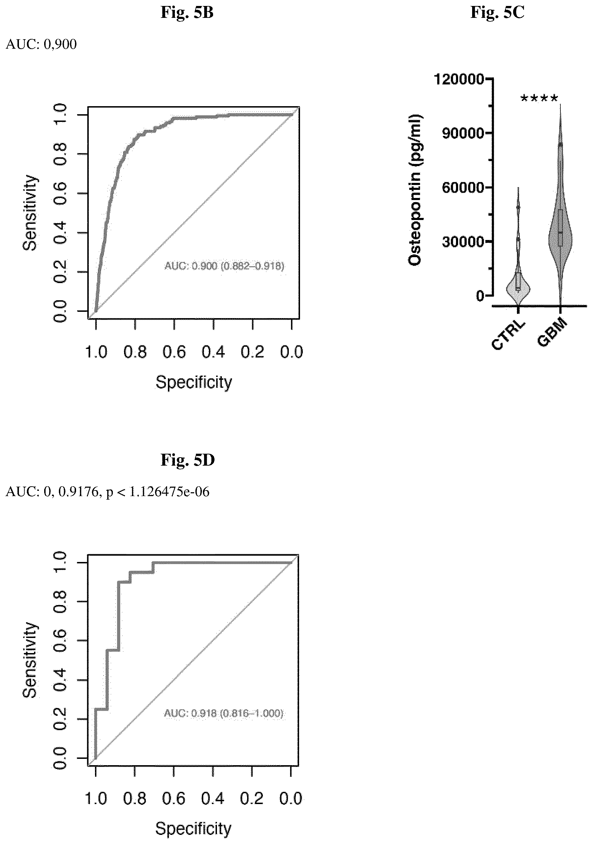

Figure 5 presents the expression level of SPP1 A) at the gene level (in tissues) and C) protein level (in blood plasma) along with the ROC analysis of the diagnostic value of a SPP1 assay as a marker at the level of B) genes among healthy brain tissues and other types of tumors and D) proteins in plasma compared to the control group of healthy subjects.

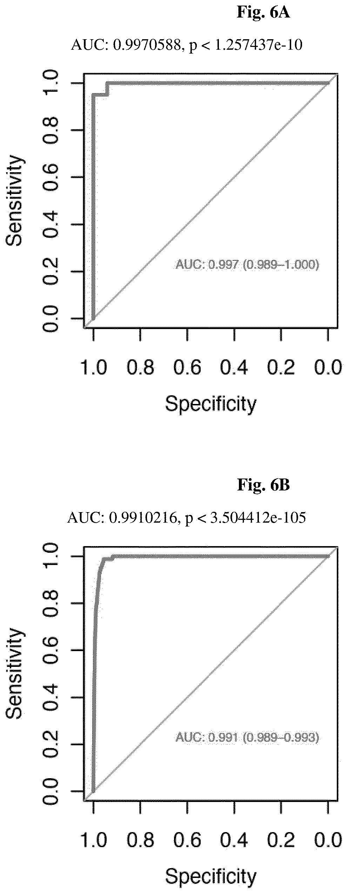

Figure 6 presents the evaluation of the diagnostic value of the combination of the MDK, CCL22, SPP1, NRGN and GFAP markers by the ROC analysis. A) evaluation of the level of these proteins in the blood plasma of patients with glioblastoma multiforme (n=20) and a control group with similar gender and age characteristics (n=18) (AUC:

0.9980588, specificity: 1, sensitivity: 0.95, p<1.257437e) and B) evaluation at the level of gene expression from RNA-Seq data of tissues of different types of tumors and healthy brain tissues (n=10909) compared to GBM tissues (n=166), (AUC=0.9910216, specificity: 0.9560913, sensitivity: 0.9879518, p ≤ 3.504412e-105).

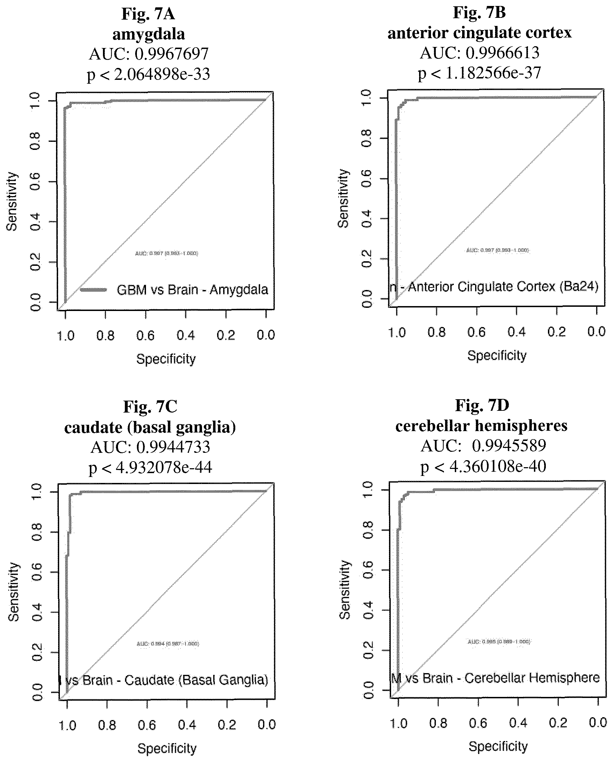

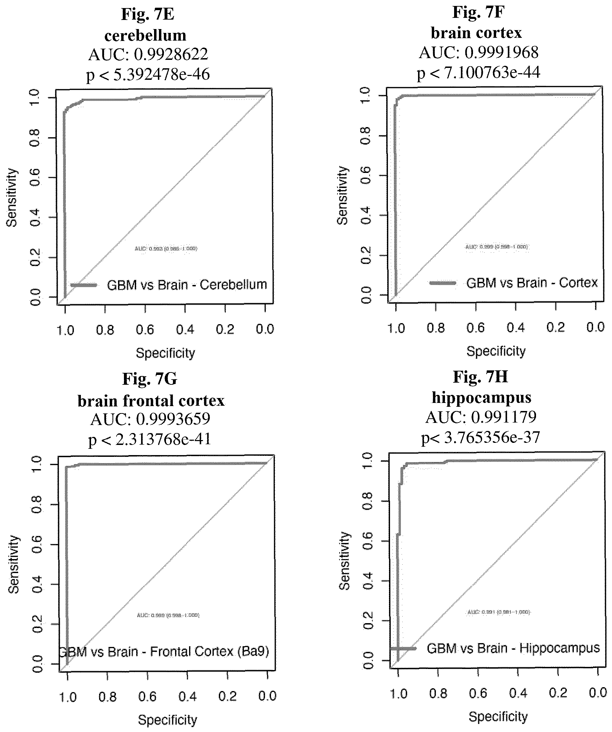

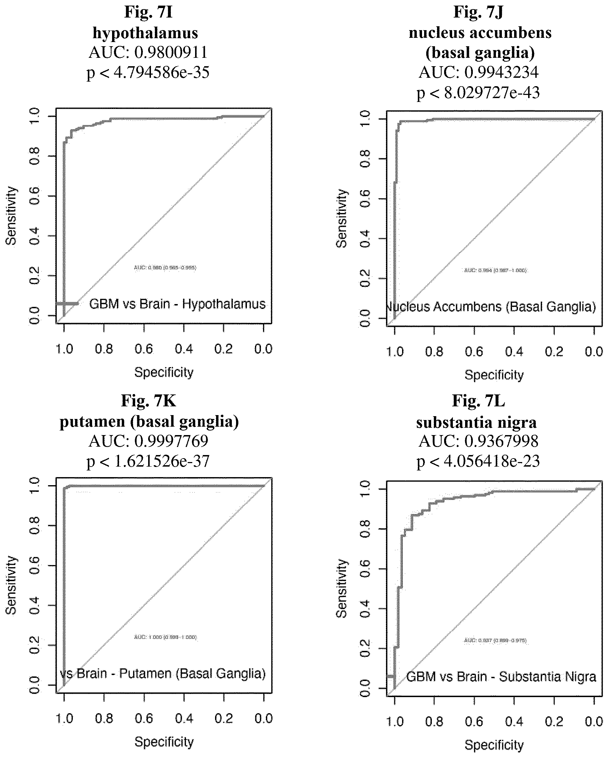

Figure 7 presents the results of the verification of the diagnostic value of GBM marker combinations in comparison with healthy brain tissues performed at the level of gene expression of bioinformatic data downloaded from publicly available TCGA and PANCAN repositories. The GBM data (n=166) with the expression data of healthy brain tissues: A) amygdala (n=69), B) anterior cingulate cortex (n=83), C) caudate nucleus (n=109), D) cerebellar hemisphere (n=93), E) cerebellum (n=119), F) brain cortex (n=105), G) frontal cortex (n=95), H) hippocampus (n=84), I) hypothalamus (n=82), J) nucleus accumbens (n=104), K) putamen (n=81), L) substantia nigra (n=57) were compared. The AUC value and statistical significance expressed as a p value are given above the graphs.

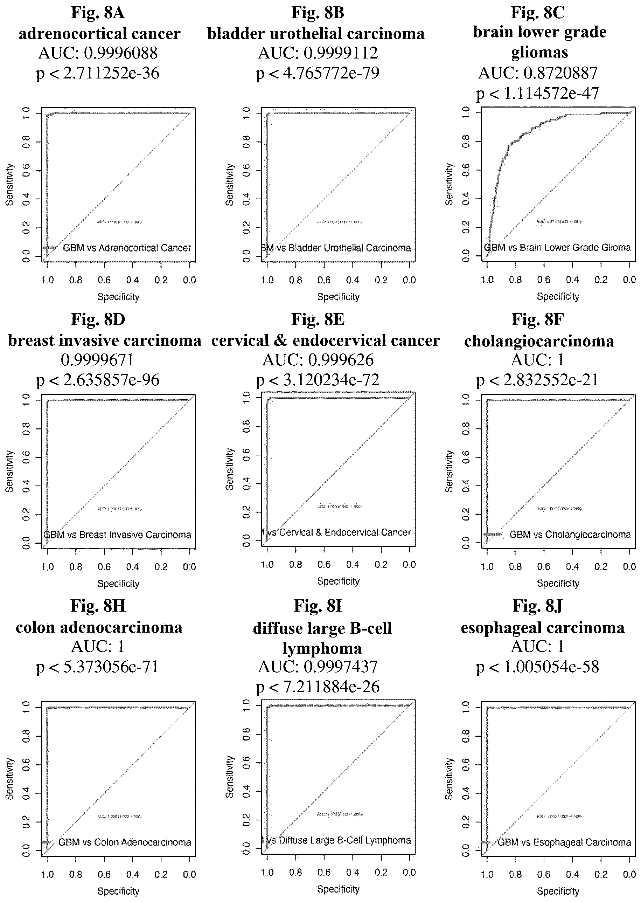

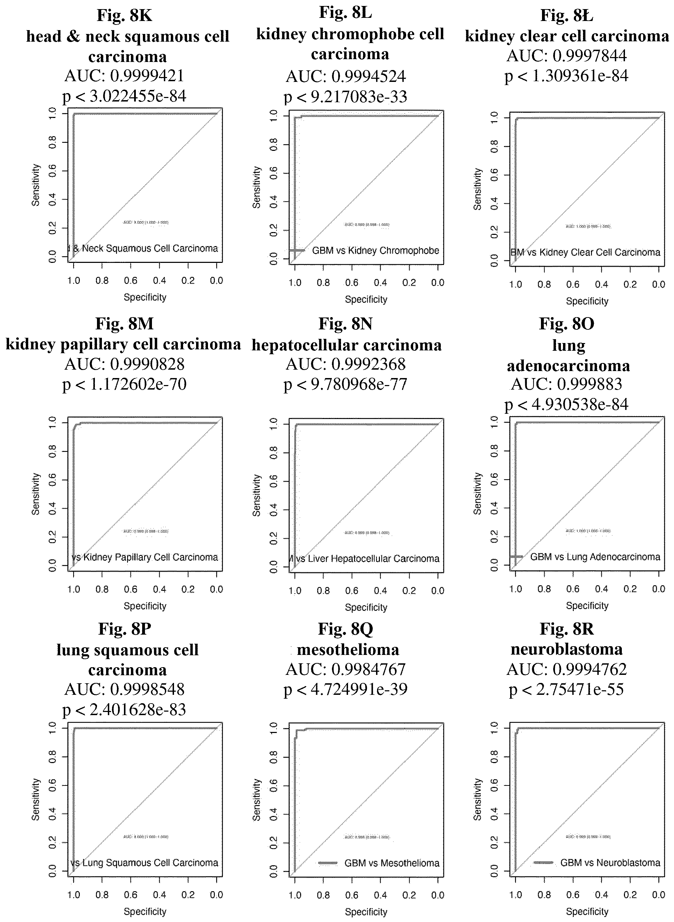

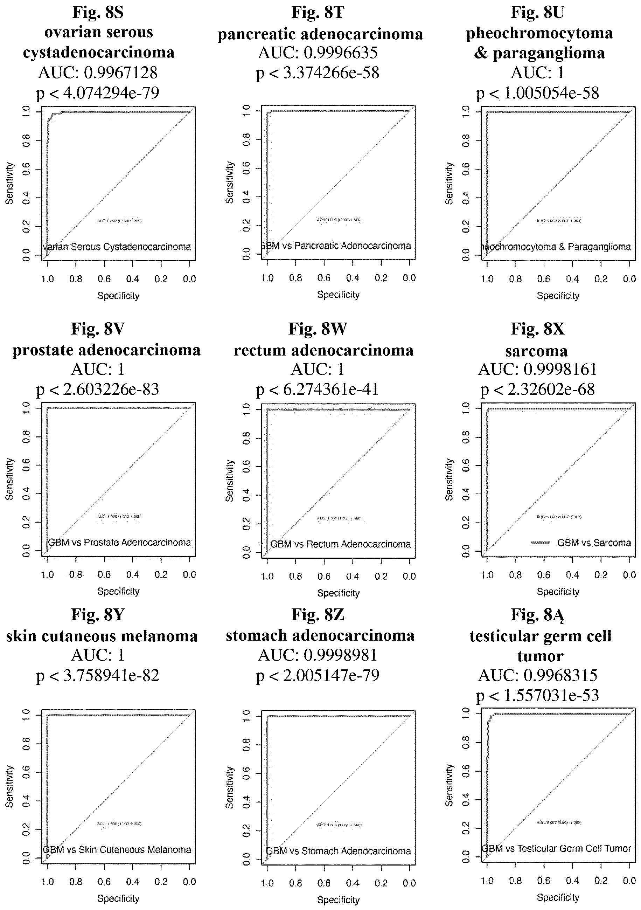

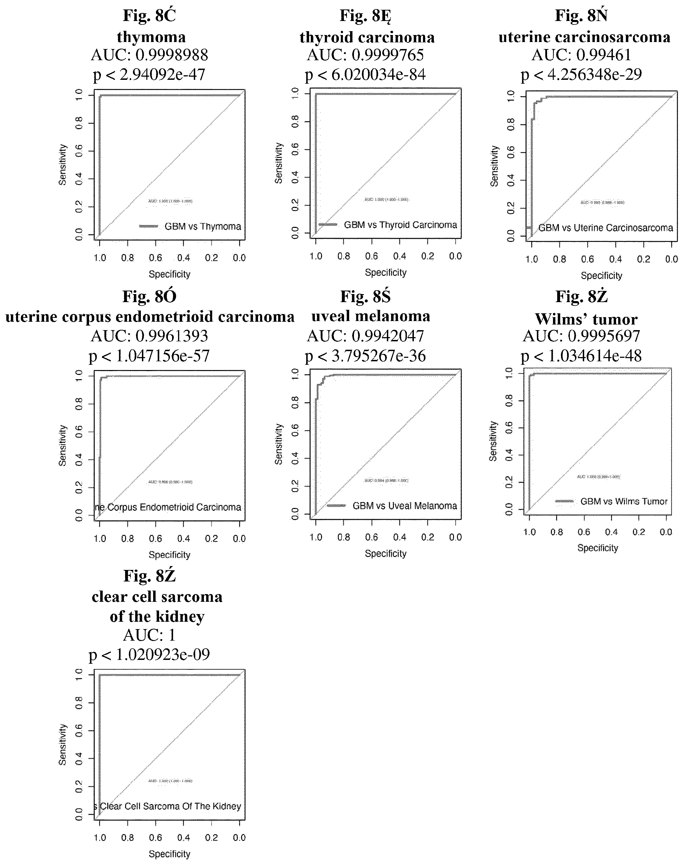

Figure 8 presents the results of the verification of the diagnostic value of GBM marker combinations in comparison with non-GBM tumor tissues. Analysis performed at the gene expression level of bioinformatic data downloaded from the publicly available TCGA and PANCAN repositories comparing data from GBM tissues (n=166) with expression data from tumor tissues of: A) adrenocortical cancer (n=77), B) bladder urothelial carcinoma (n=407), C) brain lower grade gliomas (n=523), D) breast invasive carcinoma (n=1099), E) cervical and endocervical cancer (n=306), F) cholangiocarcinoma (n=36), G) renal clear cell sarcoma (n=13), H) colon adenocarcinoma (n=290), I) diffuse large B-cell lymphoma (n=47), J) esophageal carcinoma (n=182), K) head and neck squamous cell carcinoma (n=520), L) kidney chromophobe cell carcinoma (n=66), L) kidney clear cell carcinoma (n=531), M) kidney papillary cell carcinoma (n=289), N) hepatocellular carcinoma (n=371), O) lung adenocarcinoma (n=515), P) lung squamous cell carcinoma (n=498), Q) mesothelioma (n=87), R) neuroblastoma (n=161), S) ovarian serous cystadenocarcinoma (n=427), T) pancreatic adenocarcinoma (n=179), U) pheochromocytoma and paraganglioma (n=182), V) prostate adenocarcinoma (n=496), W) rectum adenocarcinoma (n=93), X) sarcoma (n=262), Y) skin cutaneous melanoma (n=469), Z) stomach adenocarcinoma (n=414), Ą ) testicular germ cell tumor (n=154), C) thymoma (n=119), F,) thyroid carcinoma (n=512), N) uterine carcinosarcoma (n=57), Ó) uterine corpus endometrioid carcinoma (n=181), Ś) uveal melanoma (n=79), Z) Wilms' tumor (n=126), Ź) clear cell sarcoma of the kidney.

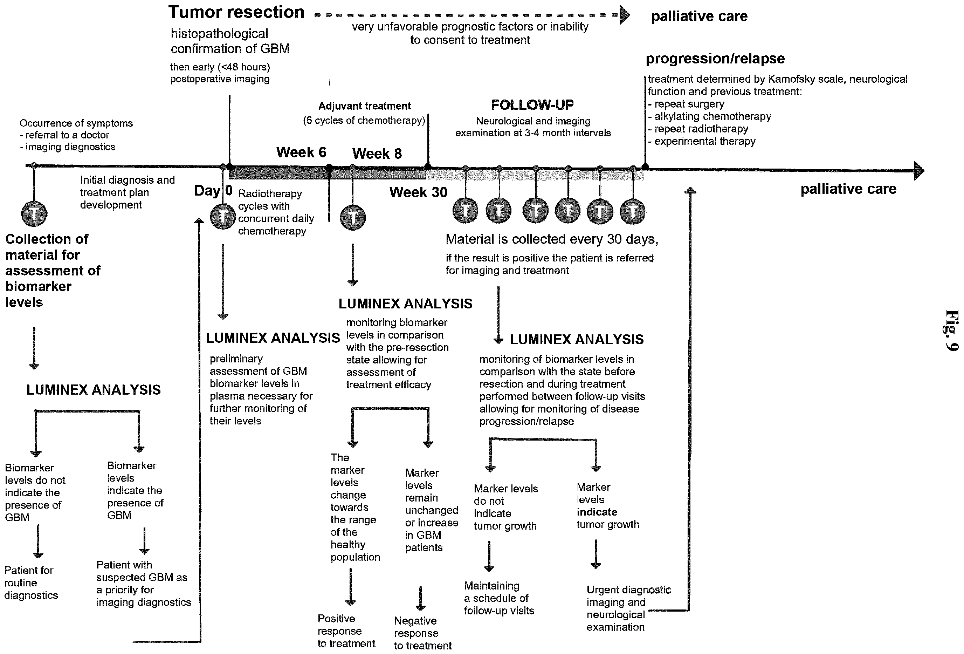

Figure 9 presents a simplified flow chart and possible scope of use of the diagnostic combination.

## Example 1. Analysis of marker levels in blood plasma and bioinformatics analysis

### 1. Methodology

#### 1.1 Human biological material

The study used 20 plasma samples from patients with histopathologically confirmed glioblastoma multiforme, which were collected before surgical resection of the tumor performed at the Department of Neurosurgery of the Medical University of Warsaw and the Voivodeship Specialist Hospital of the Medical University of Silesia in Sosnowiec. Samples were collected from 15 men and 5 women, median age 62.5 years (min. 41, max. 74, Table 1). After collecting 2 mL of peripheral blood into hematology tubes with $K_2$EDTA anticoagulant, the tubes were centrifuged for 15 min at 2000 $\times$ g (room temperature), and the obtained plasma supernatant was aliquoted into 2 Eppendorf tubes (approx. 1 mL each) and frozen at -80°C for further analysis. All procedures related to obtaining plasma samples were performed with the consent of the Bioethics Committee of the Medical University of Silesia, Ref. No. KNW/0022/KB1/2/I/17 and after receiving written informed consent from each patient, and the use of the collected samples for the purposes of this study was in accordance with the statement of the Bioethics Committee of the Medical University of Warsaw No. AKBE/168/2023. Plasma samples from 20 healthy donors with a similar age and gender proportion were purchased commercially (Catalog No. HUMANPLK2, BIOIVT). After performing Luminex analyses, two donors were excluded from the control group due to significantly elevated parameters of β-amyloid, GDNF, phosTau, YKL-40, indicating a high probability of the presence of a neurodegenerative disease. The control group contained eventually 18 donors. The characteristics of the study and control population in terms of gender and age are presented in Table 1.

Table 1. Characteristics of patients from whom blood plasma was collected for Luminex analysis.

| Control patients | | | Patients with GBM | | |
|---|---|---|---|---|---|
| sample lot | gender | age | name | gender | age |
| HMN843505 | woman | 31 | WUM 14 | woman | 57 |
| HMN843506 | woman | 56 | WUM18 | woman | 49 |
| HMN843511 | woman | 43 | WUM22 | woman | 59 |
| | | | WUM27 | woman | 66 |

(continued)

| Control patients | | | Patients with GBM | | |
|---|---|---|---|---|---|
| sample lot | gender | age | name | gender | age |
| | | | WUM29 | woman | 63 |
| | **mean** | **43.3** | | **mean** | **58.8** |
| | **median** | **43** | | **median** | **59** |
| HMN843512 | man | 36 | WUM01 | man | 62 |
| HMN843513 | man | 61 | WUM04 | man | 41 |
| HMN843514 | man | 29 | WUM07 | man | 62 |
| HMN843515 | man | 40 | WUM08 | man | 61 |
| HMN843516 | man | 73 | WUM10 | man | 48 |
| HMN843517 | man | 17 | WUM11 | man | 70 |
| HMN843518 | man | 33 | WUM17 | man | 68 |
| HMN843519 | man | 64 | WUM20 | man | 51 |
| HMN843520 | man | 57 | WUM21 | man | 68 |
| HMN843521 | man | 64 | WUM24 | man | 71 |
| HMN843522 | man | 71 | WUM25 | man | 57 |
| HMN843523 | man | 22 | WUM26 | man | 61 |
| HMN843524 | man | 49 | WUM28 | man | 70 |
| HMN843525 | man | 56 | WUM34 | man | 63 |
| HMN843526 | man | 63 | WUM35 | man | 69 |
| | **mean** | **49** | | **mean** | **61.5** |
| | **median** | **56** | | **median** | **62** |
| **Mean of all** | | **48.1** | **Mean of all** | | **60.8** |
| **Median of all** | | **52.5** | **Median of all** | | **62** |

*1.2 Analysis of marker levels in blood plasma using the Luminex method*

Quantitative assessment of plasma levels of CCL22, GFAP, MDK, NRGN, and SPP1 was performed by the LUMINEX method (Luminex200 xMAP Technology system, LUMINEX corp.), using the Human Luminex Discovery Assay kit (#LXSAHM, R&D Systems) according to the manufacturer's protocol. Immediately prior to analysis, samples were centrifuged at 16,000 $\times$ g for 4 min to get rid of artifacts. Plasma samples were not diluted for analysis. The marker levels were determined in plasma from 20 patients with confirmed glioblastoma multiforme and from 20 healthy subjects with similar gender and age proportions (commercially purchased from BIOIVT).

Statistical calculations were performed using R 4.3.1 (2023-06-16) package. The "tidyverse" (2.0.0) software was used to prepare all figures. Averaged results (pg/mL) exported from the Luminex software were imported to the R software. The concentration of Midkine was above the limit of detection in one sample and was set at the limit of detection (8,670 pg/mL). The concentrations of CXCL2 and CCL22 were below the limit of detection in one sample and were set at the lower limit of detection. The concentration of each analyte in the samples was visualized as a heat map using ComplexHeatmap (2.16.0). The concentration of each analyte in healthy serum and from glioma patient samples was visualized and compared in a violin plot type diagram with the Wilcoxon test using "ggplot2" (3.4.2). The potential diagnostic accuracy of the expression of each of the proteins of interest individually (MDK, CCL22, SPP1, NRGN, and GFAP) as well as the combination of all five proteins included in the linear regression model was then assessed using receiver operating characteristic (ROC) curve analysis. The ROC curves were calculated and drawn using the "pROC" (1.18.4) package to assess the sensitivity and specificity of the risk score for diagnostic accuracy. The area under the curve (AUC) was used as a measure of prognostic accuracy.

*1.3 Bioinformatics data analysis*

The study used large-scale gene expression analysis using publicly available RNA sequencing data of glioblastoma multiforme tumor fragments collected from major global repositories. Statistical calculations were performed using the R 4.3.1 (2023-06-16) package. The "tidyverse" (2.0.0) software was used to prepare all figures. Gene expression profiles (normalized RSEM numbers) of normal and tumor tissues from 19,120 patients, along with their clinical data, were downloaded from TCGA Xena Hub (https://tcga.xenahubs.net) with the cohort name: TCGA TARGET GTEx (access date 2023-07-07). Data were filtered for the expression of five genes: *MDK, CCL22, SPP1, NRGN,* and *GFAP.* Only samples from solid tumors (9934 tumors) and healthy brain tissues (1141 samples) were included in the analysis. The expression of each selected gene was visualized as a violin plot type diagram in different tumor types and healthy brain tissues using the "ggplot2" package (3.4.2). The potential diagnostic value of the expression of each gene separately and in combination with all five genes included in the linear regression model was then assessed using ROC analysis. The accuracy of the combination of all five genes was assessed for all sample categories combined, as well as for each tumor type and healthy tissue separately. ROC curves were calculated and drawn using the "pROC" (1.18.4) package to assess the sensitivity and specificity of the risk score for diagnostic accuracy. The area under the curve (AUC) was used as a measure of diagnostic accuracy.

### 2. Description of marker combinations and interpretation of results

As a result of the analyses conducted, a unique combination of five biological markers found in blood plasma was obtained: CCL22, GFAP, MDK, NRGN, SPP1, whose simultaneous determination in a plasma sample enables low-invasive screening diagnostics of glioblastoma multiforme. The set of these markers is not obvious to a person skilled in the art, because it includes proteins of completely different biological nature, which, although they are produced by GBM tumor tissues and their level is measurable in blood plasma, there are no reports or theoretical indications that they may be an unambiguous determinant of the occurrence of glioblastoma multiforme. Glioblastoma multiforme is a brain tumor, and due to the existence of the blood-brain barrier and selective transport of substances between the blood and the cerebrospinal fluid (and vice versa), it is not obvious whether substances produced in the brain will also be present in the blood, and whether, due to the extensiveness of the vascular bed, they will be present in measurable quantities. The second challenge in the process of selecting markers associated with brain tumors is the lack of methods that allow for safe acquisition from the patient or *in situ examination* of these tissues. Therefore, most of the data collected on the biology of GBM is related to the analysis of material from tumor resection, which was previously disturbed for the purposes of histopathological examination. Concerning such material, the structure of the neoplastic lesion can be described relatively well, but it is very difficult to draw conclusions regarding more dynamic processes, such as its secretory activity. In this case, gene transcription studies using genome sequencing techniques have proven invaluable. They allowed not only the insight into the expression level of thousands of genes in cancer cells, but also identification of cell types found in the cancer tissue. Although transcription data do not translate linearly into the production or secretion of proteins by cells, they are still the main source of information on GBM biology. These very data were used for the initial screening to identify potential markers of glioblastoma multiforme. To conduct this analysis, both bioinformatic data and transcriptome of GBM tumors, as well as healthy brain tissues, and data from other types of tumors were used. All calculations regarding diagnostic sensitivity and specificity in the ROC analyses of the markers mentioned, and their combinations, aimed at the identification of people with GBM were performed both on the bioinformatic data and on the absolute values, i.e. the values of determined concentrations of the markers in blood plasma expressed in [pg/mL], where in the practical implementation of the invention the concentration values were determined using the precise Luminex method, the use of which determines the reliability of the obtained results and the universality of their use.

### 2.1 Analysis of the diagnostic potential of individual markers

Within the indicated combination of the five markers characteristic for GBM, three of them, MDK, GFAP and SPP1, were previously mentioned in the scientific literature and associated with the development of glioblastoma multiforme, but their use as stand-alone diagnostic markers was shown to be ineffective. Midkine (MDK) is a pleiotropic growth factor active mainly during prenatal human development, which in a healthy adult organism is expressed at a very low level, and undergoes high overexpression in pathological conditions, including ischemia, inflammation, autoimmune processes, or in many cancers [Jones, D.R., Measuring midkine: the utility of midkine as a biomarker in cancer and other diseases. Br J Pharmacol, 2014. 171(12): p. 2925-39.] Many studies have already been performed to determine the concentration of midkine in the blood (most often in serum) of healthy subjects, and regardless of the diversity of the studied populations, research methods, and approach to testing, the data are relatively consistent regarding the physiological range of midkine concentrations in the blood below 625 pg/mL in 95% of the examined subjects [Jones, D.R., 2014, *supra].* The concentration of midkine in the blood significantly increases in many tumor diseases, ischemic diseases, kidney damage, autoimmune diseases, sepsis, Alzheimer's disease, which does not make it a marker specific for one disease, which is also confirmed by the bioinformatic analyses of midkine expression at the gene level presented hereinbelow (Fig. 1). In oncology alone, the determination of midkine in blood does not allow for differentiation between types of tumor [Jones, D.R., 2014, *supra].* The results presented herein also confirmed the poor diagnostic value of midkine when tested alone, in both gene expression data analysis (AUC: 0.538, Fig. 1B), as well as in terms of the absolute values of concentration thereof in blood plasma (AUC: 0.850, Fig. 1D). The dynamics of concentrations is how the increased levels of midkine in blood in the emergency cases (acute kidney injury, ischemic conditions) can be distinguished from neoplastic diseases, because in emergencies the concentration of midkine decreases relatively quickly (usually within a few days), while in neoplastic diseases it usually increases progressively until the resection of the neoplastic lesion (summarized in Krzystek-Korpacka, M. and M. Matusiewicz, Circulating Midkine in Malignant, Inflammatory, and Infectious Diseases: A Systematic Review, in Midkine: From Embryogenesis to Pathogenesis and Therapy, M. Ergüven, T. Muramatsu, and A. Bilir, Editors. 2012, Springer Netherlands: Dordrecht. p. 69-85.).

Despite the fact that midkine is not a good diagnostic marker on its own, there are several reasons why it plays an important role in the newly developed diagnostic marker combination. First and foremost, among the five selected markers, midkine is the most universal indicator of the presence of a pathological process in the patient. After excluding any past or present comorbidities in the medical history, its elevated concentration in blood plasma strongly indicates the activity of a neoplastic process. Moreover, the analysis of bioinformatic data showed a very consistent pattern of its expression in glioblastoma multiforme, proportionally increasing with the classification of gliomas, and correlating with reduced survival [Cheng, Y.P., et al., Midkine high-grade expression gliomas: Correlation of this novel marker with proliferation and survival in human gliomas. Surg Neurol Int, 2014. 5: p. 78.] The fact of its stable and measurable (in hundreds of pg/mL) expression in the blood plasma of the healthy population is also significant. Nevertheless, in order to strengthen the diagnostic value of midkine in glioblastoma multiforme, it turned out to be necessary to add further, more specific markers, among which one of the most important is GFAP.

GFAP *(glial fibrillary acidic protein)* is a structural protein of astrocytes - a type III intermediate filament expressed in mature astrocytes of the gray and white matter, cerebellum, subventricular and subgranular zones and in Mueller cells in the retina [Middeldorp, J. and E.M. Hol, GFAP in health and disease. Threshold Neurobiol, 2011. 93(3): p. 421-43.]. It is therefore a marker characteristic of the central nervous system. Although the issue of GFAP protein entry into the circulation is not yet fully explained, it is most likely transferred along a bidirectional exchange both through the blood-brain barrier (and blood-cerebrospinal fluid), at the level of the arachnoid villi and along the flow through the lymphatic system and cervical lymph nodes [Plog, B.A., et al., Biomarkers of traumatic injury are transported from brain to blood via the glymphatic system. J Neurosci, 2015. 35(2): p. 518-26., Abdelhak, A., et al., Blood GFAP as an emerging biomarker in brain and spinal cord disorders. Nat Rev Neurol, 2022. 18(3): p. 158-172.]. Blood GFAP levels have been shown to reflect the clinical severity and extent of intracranial pathology after traumatic brain injury, which is why in 2018 the FDA approved a blood diagnostic test for GFAP and UCH-L1 for clinical use in mild traumatic brain injury [Abdelhak, A., et al., 2022, *supra*]. Increasing evidence supports the potential clinical use of blood GFAP testing in neuroinflammatory and neurodegenerative diseases, and in any condition in which the CNS is involved in systemic diseases. Increased levels of GFAP in the blood of patients are also noted in brain tumors, especially glioblastoma multiforme. The present study confirmed that the level of GFAP in the blood of subjects with glioblastoma multiforme is significantly higher than in healthy subjects (where this level was at the borderline of quantification) (Fig. 2C). Moreover, the analysis of the bioinformatic data showed that this marker was specifically expressed in brain tissues, and its expression in other types of tumors was at a much lower level (Fig. 2A). Literature data also indicate that its level in the blood of people with GBM is higher than in those with primary brain tumors other than glioblastomas and people with brain metastases, but it does not allow to distinguish between GBM and gliomas of a lower malignancy grade (summarized in Abdelhak, A., et al., 2022, *supra*). There is evidence that the GFAP concentration in the blood of GBM patients correlates with preoperative tumor volume, tumor necrosis volume and GFAP expression levels in the tumor tissue [Tichy, J., et al., Prospective evaluation of serum glial fibrillar acidic protein (GFAP) as a diagnostic marker for glioblastoma. J Neurooncol, 2016. 126(2): p. 361-9].

Although GFAP seems to be a relatively well-studied protein, there are still some limitations to its use as a diagnostic marker. First of all, there are no detailed studies on the range of its physiological concentrations in the blood of healthy people in an age-differentiated population (it is known that its expression at the mRNA level in the brain increases with age [Nichols, N.R., et al., GFAP mRNA increases with age in rat and human brain. Neurobiol Aging, 1993. 14(5): p. 421-9.]), and secondly, most of the literature data available to date are based on ELISA tests, the sensitivity and resolution of which are worse than those of highly sensitive bead-based tests (such as Luminex) or mass spectrometry. Moreover, there is currently no data on the dynamics of GFAP levels in different diseases, and the balance between GFAP release and its clearance is not yet well defined. Nevertheless, regardless of its diagnostic value as a single marker, GFAP plays a key role in the combination of markers of the invention, enabling the distinction of GBM from non-CNS tumors.

The marker combination according to the invention includes another marker closely related to the CNS - neurogranin (NRGN). It is a small protein involved in signal transduction in postsynaptic neurons and in the dendrites of pyramidal neurons of different layers of the cerebral cortex, participating in the formation of synaptic plasticity and regeneration, as well as long-term synaptic potentiation via calcium and calmodulin signaling pathways [Xiang, Y., et al., Neurogranin: A Potential Biomarker of Neurological and Mental Diseases. Front Aging Neurosci, 2020. 12: p. 584743.]. Increased levels of neurogranin in the cerebrospinal fluid have been described in the course of neurological and psychiatric diseases (including Alzheimer's disease, Parkinson's disease, Creutzfeldt -Jakob disease, Huntington's disease, post-traumatic brain injury, moderate cognitive impairment, dementia, schizophrenia, depression, *etc.,* as summarized in Xiang, Y., et al., 2020, *supra*). Neurogranin is most often studied in the cerebrospinal fluid and, like GFAP, mainly by ELISA tests. There are relatively few studies determining its level in the blood and they mainly concern Alzheimer's disease and acute ischemic stroke. Increased levels of NRGN in the blood most likely result from a decrease in the tightness of the blood-brain barrier. It has been shown that, in contrast to the cerebrospinal fluid determinations, the NRGN determination in blood has a low diagnostic potential for Alzheimer's disease [De Vos, A., et al., C-terminal neurogranin is increased in cerebrospinal fluid but unchanged in plasma in Alzheimer's disease. Alzheimers Dement, 2015. 11(12): p. 1461-1469, Kvartsberg, H., et al., Characterization of the postsynaptic protein neurogranin in paired cerebrospinal fluid and plasma samples from Alzheimer's disease patients and healthy controls. Alzheimers Res Ther, 2015. 7(1): p. 40.], and the data on acute ischemic stroke are contradictory (one study proved that the level of NRGN in blood has a diagnostic value in the first 24 hours after the occurrence of acute ischemic stroke compared to the NRGN level in the healthy population, but it has no prognostic value [Kusdogan, M., et al., The diagnostic and prognostic value of serum neurogranin in acute ischemic stroke. J Stroke Cerebrovasc Dis, 2023. 32(2): p. 106889.], and in the other one, that its level in the blood correlates only with the size of the stroke, and is not a potentially good marker for acute ischemic stroke [De Vos, A., et al., 2015, *supra]*). Nevertheless, data on neurogranin in patients with glioblastoma multiforme is very scarce, mainly concerning bioinformatic analyses of its reduced expression in GBM tissues compared to the healthy brain tissue [Yokota, T., et al., Identification of histological markers for malignant glioma by genome-wide expression analysis: dynein, alpha -PIX and sorcin. Acta Neuropathol, 2006. 111(1): p. 29-38.]. There are also reports in the literature that NRGN is not a GBM-specific protein, as its level in the blood of patients with gliomas is lower than in the control population [Lange, R.P., et al., Evaluation of eight plasma proteins as candidate blood based biomarkers for malignant gliomas. Cancer Invest, 2014. 32(8): p. 423-9.]. In the studies presented in the publication by Lange, R.P., et al., 2014, NRGN was determined using a proprietary ELISA test (the method for the preparation of which having not been described), and the NRGN protein standard was the human recombinant NRGN protein produced by the authors of the publication in a bacterial system. Therefore, the results of this analysis are highly questionable and unreproducible. The bioinformatic analysis, the results of which are presented herein, similarly to the literature sources, showed a lower expression of the *NRGN* gene in GBM tumors compared to the expression in healthy brain tissues (especially the cerebral cortex, Fig. 3A), while the level of the NRGN protein in the blood plasma of patients with glioblastoma multiforme turned out to be markedly higher than in healthy subjects (Fig. 3C). It should also be emphasized that the studies described herein were performed using a commercially available, manufacturer-validated Luminex kit, so our results from these studies are much more reliable. Due to the discrepancy between expression at the gene level and the amount of protein in biological fluids, which is not unusual especially for proteins whose secretion is dynamic and responsive, as in the case of NRGN (it is produced and stored in the cell and secreted only in response to stimuli), much better ROC analysis results were obtained for the data at the protein level, which resulted in a very high AUC = 0.997 (Fig. 3D) as compared with the gene expression data (AUC = 0.790, Fig. 3B).

In order to increase the diagnostic value of the marker combination according to the invention, the diagnostic panel also includes two additional proteins. The first of them, the chemokine CCL22, is a key mediator of leukocyte trafficking in chronic inflammatory processes, allergies and tumors. It is not a marker specific for GBM, as it is produced mainly by dendritic cells, macrophages, but also many types of tumor cells, by acting *via* the CCR type 4 receptor (CCR4), is responsible for attracting mainly monocytes, Th2 cells and subsets of regulatory T lymphocytes [Anz, D., et al., Suppression of intratumoral CCL22 by type and interferon inhibitions migration of regulatory T cells and blocks cancer progression. Cancer Res, 2015. 75(21): p. 4483-93.] In addition to its role in inflammation, CCL22 is constitutively expressed in lymphoid organs, where it controls immune processes by recruiting regulatory T cells to dendritic cells [Rapp, M., et al., CCL22 controls immunity by promoting regulatory T cell communication with dendritic cells in lymph nodes. J Exp Med, 2019. 216(5): p. 1170-1181.] Thus, CCL22 plays a role of a checkpoint in the immune system, regulating the balance between tolerance and immune activation [Rohrle, N., M.M.L. Knott, and D. Anz, CCL22 Signaling in the Tumor Environment. Adv Exp Med Biol, 2020. 1231: p. 79-96.] It is interesting to note that while in a large proportion of tumors (including B-CLL, Hodgkin lymphoma, breast, lung and gastrointestinal cancers), the level of CCL22 in the blood is elevated compared to the level in the healthy population (summarized in Wiedemann, GM, et al., Cancer cell-derived IL-1alpha induces CCL22 and the recruitment of regulatory T cells. Oncoimmunology, 2016. 5(9): p. e1175794.), in GBM, as shown herein, the level of CCL22 decreases very significantly (Fig. 4C). Currently, it is difficult to precisely determine the mechanism associated with this decrease, since, although gene expression analysis indicates that CCL22 is expressed in tumor tissues (and confirms that the expression in GBM is one of the lowest among tumors), the expression in GBM tissues is still higher than in healthy brain tissues (Fig. 4A).

This peculiar decrease in CCL22 levels in the blood of glioma patients has been described so far only in one paper, which showed a significantly reduced CCL22 level in the serum of glioma patients compared to a healthy control group (1208 glioma cases and 976 control sera), which positively correlated with CD4+ cell count and survival time independently of age, histology, stage and IDH mutation status [Zhou, M., et al., Serum macrophage-derived chemokine/CCL22 levels are associated with glioma risk, CD4 T cell lymphopenia and survival time. Int J Cancer, 2015. 137(4): p. 826-36.] The decrease in CCL22 levels in blood in other diseases has been described so far only in the rare fragile X syndrome (FXS) [Van Dijck, A., et al., Reduced serum levels of pro-inflammatory chemokines in fragile X syndrome. BMC Neurol, 2020. 20(1): p. 138.], and rheumatoid arthritis [Skrzypkowska, M., et al., Cytokines and chemokines multiplex analysis in patients with low disease activity rheumatoid arthritis. Rheumatol Int, 2022. 42(4): p. 609-619.], however, it was not as significant as in GBM. Due to this characteristic, *i.e.* the opposite dynamics than in the case of the rest of the markers (decrease rather than increase in the level of circulating CCL22, which in itself has good diagnostic parameters, AUC = 0.909) and high specificity of the decrease in connection with gliomas, CCL22 is a very important marker of the combination of markers according to the invention.

The last marker that has been included in the marker combination according to the invention is osteopontin (gene symbol *SPP1*). It is a non-structural extracellular matrix protein produced by multiple types of cells (including epithelium, endothelium, fibroblasts, hepatocytes, lens cells, T lymphocytes, B lymphocytes, macrophages, NK cells, NKT cells, neurons, glial cells, Schwann cells, osteoblasts, osteoclasts and vascular smooth muscle cells [Kahles, F., H.M. Findeisen, and D. Bruemmer, Osteopontin: A novel regulator at the cross roads of inflammation, obesity and diabetes). Molecular metabolism, 2014. 3(4): p. 384-393.]), physiologically involved in the processes of adhesion, proliferation, differentiation, migration and immunomodulation (summarized in Kariya, Y. and Y. Kariya, Osteopontin in Cancer: Mechanisms and Therapeutic Targets. International Journal of Translational Medicine, 2022. 2(3): p. 419-447.). Osteopontin also plays a role in a number of pathological processes ranging from those related to inflammation and tissue fibrosis, autoimmune processes, to carcinogenesis and metastasis of many types of tumors (skin, head and neck, thyroid, breast, lung, esophagus, stomach, liver, pancreas, colon, kidney, bladder, prostate, ovary cancers, melanoma, myeloma, osteosarcoma and glioblastoma multiforme) [Kariya, Y. and Y. Kariya, 2022, *supra].* The studies described herein on the expression of *SPP1* in tumor and healthy brain tissues showed that in GBM, closely following papillary renal carcinoma (kidney papillary cell carcinoma), it is expressed to the highest extent (Fig. 5A). Analysis of osteopontin levels in blood plasma showed that high expression at the gene level is also reflected in the circulating protein level, and this level reaches very high values in patients with GBM (Fig. 5C).

Literature data confirm that increased *SPP1* expression in GBM is correlated with a shorter survival time of glioma patients, and high SPP1 level in their blood was associated with a worse prognosis [Sreekanthreddy, P., et al., Identification of potential serum biomarkers of glioblastoma: serum osteopontin levels correlate with poor prognosis. Cancer Epidemiol Biomarkers Prev, 2010.19(6): p. 1409-22.] Although osteopontin is not a glioma-specific protein, the present inventors decided to include SPP1 in the marker combination of the invention because of its potential in monitoring tumor progression and recurrence. Based on the results of patients with early stage lung adenocarcinoma, where a significant decrease in plasma SPP1 levels was observed after a surgical resection of the tumor and a significant re-increase in those with tumor recurrence after the surgery [Blasberg, JD, et al., Reduction of elevated plasma osteopontin levels with resection of non-small-cell lung cancer. J Clin Oncol, 2010. 28(6): p. 936-41.], it can be assumed that SPP1 has the potential as a tool for monitoring cancer progression and detecting tumor recurrence after treatment [Hao, C., et al., Human osteopontin: Potential clinical applications in cancer (Review). Int J Mol Med, 2017. 39(6): p. 1327-1337.]. Moreover, similar results have been described for other types of tumors; increased levels of SPP1 in plasma and tumor tissues have been identified in patients with breast cancer and have been shown to be associated with the presence of metastases and reduced survival time of patients [Anborgh, PH, et al., Role of plasma osteopontin as a biomarker in locally advanced breast cancer. Am J Transl Res, 2015. 7(4): p. 723-32.] In patients with pancreatic ductal adenocarcinoma, serum osteopontin levels have been shown to increase with the stage of the tumor [Koopmann, J., et al., Evaluation of osteopontin as a biomarker for pancreatic adenocarcinoma. Cancer Epidemiol Biomarkers Prev, 2004. 13(3): p. 487-91.]. Elevated blood osteopontin levels were also strongly associated with increased stage, grade, and tumor size in melanoma patients [Kiss, T., et al., The role of osteopontin expression in melanoma progression. Tumour Biol, 2015. 36(10): p. 7841-7.]. In summary, osteopontin in the marker combination according to the invention seems to have potential in the assessment of cancer progression and the results of its treatment.

## 2.2 Analysis of the diagnostic potential of the marker combinations

The data presented above for individual markers were an introduction to the establishment of marker combinations enabling the diagnosis of glioblastoma multiforme. Although some of the markers had relatively high independent diagnostic parameters (for example, when their levels in plasma were compared to the levels in samples from the healthy population), none of them is specific enough for GBM to be used as a single marker. Collecting them into a combination of markers, determined using the Luminex method simultaneously in one small (25-50 μl) amount of plasma sample, allowed to obtain extremely promising diagnostic parameters for this combination, i.e. a high AUC index: 0.9970588 (0.989 - 1.000), with a significance level of $p < 1.257437e-10$ for the comparison of plasma samples from patients with GBM to control donors (Fig. 6A). The analysis of the diagnostic value of this combination at the level of gene expression reached the values of AUC: 0.9910216 (0.989 - 0.993), specificity: 0.9560913, sensitivity: 0.9879518, with a significance level of $p < 3.504412e-105$ (Fig. 6B), for the comparison of the expression data of GBM tissues (n=166) to the expression in healthy brain tissues and tissues of other 33 types of tumors (n=10909).

The verification of the diagnostic value of the marker combination according to the invention was performed at the level of gene expression in comparison with healthy brain tissues (Fig. 7). Due to the fact that the Inventors did not have blood samples from patients with other types of tumors, the verification of the diagnostic value of the marker combination in cancers other than GBM was also performed at the level of gene expression (Fig. 8). The results of the ROC analysis of the diagnostic value of the combination in comparison with healthy brain tissues showed that in the case of healthy brain tissues, on the basis of the combination of the five markers according to the invention, GBM can be distinguished from healthy tissue with very high sensitivity and specificity.

Verification of the combination of the five markers according to the invention at the level of gene expression among 32 types of tumors other than gliomas also showed extremely good diagnostic parameters, reaching in the worst case still a very high AUC value: 0.994 (0.965 - 1.000) for ocular melanoma (Fig. 8Ś). The lowest diagnostic value of our marker combination based on ROC analysis, as expected, was noted for the comparison of glioblastoma multiforme to gliomas of lower malignancy, whose AUC value: 0.872 (0.843 - 0.901) (Fig. 8C). However, this result is still very high and does not reduce the potential for using the combination of markers according to the invention in the diagnostic procedure of brain tumors. Moreover, the results presented in comparison with lower-grade gliomas confirm that the markers constituting the essence of the invention constitute a signature for glia proliferation.

In summary, the verification at the level of gene expression of the combination of the five markers according to the invention for GBM: MDK, CCL22, SPP1, NRGN and GFAP, created by the Inventors, showed its extremely high diagnostic sensitivity and specificity in comparison with healthy brain tissues, as well as tissues of 33 types of tumors other than GBM.

**Example 2 Determining the presence or absence of GBM in a patient**

Based on the results presented in Example 1, the risk coefficient "Y" was determined, which allows to classify whether the sample comes from a healthy subject or a person suffering from GBM, according to the following formula:

$$Y = Midkine\left[\frac{pg}{mL}\right] * 2.311382e^{-05} + Osteopontin\left[\frac{pg}{mL}\right] * 5.020056e^{-06} +$$

$$Neurogranin\left[\frac{pg}{mL}\right] * 7.851757e^{-04} + CCL22\left[\frac{pg}{mL}\right] * -5.500833e^{-04} + GFAP\left[\frac{pg}{mL}\right] * -3,058356e^{-04}$$

Absolute concentrations (pg/mL) of five different proteins were measured by Luminex in the patients' serum. To investigate the relationship between these protein concentrations and the patients' status in the control or treatment group, multiple linear regression analysis was used using the lm() function in the R language. Regression coefficients, including the intercept and coefficients for individual proteins, were estimated using this function. The least squares algorithm was used to best fit the model to describe the relationship between the independent variables (protein concentrations) and the dependent variable (patient status). The values of these coefficients were adjusted to minimize the sum of squared differences between the predicted and actual values of the dependent variable.

With a set cut-off point for the risk factor 'Y' equal to 0.2472802 (at which all samples for which the calculated risk factor based on the determined concentration values of all the CCL22, GFAP, MDK, NRGN and SPP1 markers in the sample is less than the cut-off point are classified as samples originating/collected from healthy subjects, and those for which the calculated risk factor "Y" is equal to or greater than the cut-off point are classified as samples originating/collected from subjects with glioblastoma multiforme), the test achieves 100% specificity and 95% sensitivity.

**Example 3. A course of action and scope of use of the marker combinations according to the invention**

If the patient experiences symptoms such as, for example, headache, nausea and vomiting, deterioration of brain function (e.g. problems with thinking and understanding), memory loss, personality changes, vision problems, speech difficulties, epileptic seizures, characteristic of GBM and other neurological conditions, a blood sample is taken from the patient and the levels of the combination of markers MDK, CCL22, SPP1, NRGN and GFAP are determined. If the level of the combination of markers does not indicate the presence of GBM, the patient is referred for routine diagnostics. If the level of markers indicates the presence of GBM, the patient is referred for urgent diagnostic imaging with suspicion of GBM. The patient is then referred for resection of the tumor and then subjected to radiotherapy and chemotherapy. After resection, before starting therapy and during therapy, the levels of the combination of markers MDK, CCL22, SPP1, NRGN and GFAP are determined. Monitoring the level of markers allows for the assessment of the effectiveness of the treatment. On this basis, a decision can be made about adjuvant treatment (e.g. chemotherapy).

After the end of treatment, a blood sample is taken from the patient periodically and the level of the MDK, CCL22, SPP1, NRGN and GFAP markers is determined. The comparison of the level of these markers to the level before resection and during treatment allows for the assessment of disease progression or relapse. Marker determinations are performed, for example, at 30-day intervals. At the same time, during this period, neurological and imaging tests are performed at 3-4 month intervals. If a change in marker levels is noted in the direction of tumor growth, the patient is referred for imaging diagnostics and a neurological examination, which will allow for further therapeutic steps or palliative care.

Determination of the markers in a patient sample at each stage of diagnosis and monitoring is performed using the Luminex analysis.

A simplified procedure flowchart and possible scope of use of the diagnostic combination are shown in Fig. 9.

**Claims**

1.   A method of diagnosing glioblastoma multiforme in a subject, comprising

(a) a step of determining concentrations of a combination of the following five markers: CCL22, GFAP, MDK, NRGN and SPP1 in a sample collected from the said subject, and
(b) a step of determining the risk factor using the concentrations determined in step (a), comparing it with the predetermined cut-off point and determining on this basis the presence or absence of glioblastoma multiforme in the subject

wherein the cut-off point is determined on the basis of concentrations determined in samples collected from healthy subjects, samples collected from subjects suffering from glioblastoma multiforme, and/or samples collected from subjects suffering from another type of tumor.

2.   A method of monitoring a subject's response to treatment for glioblastoma multiforme, comprising

(a) a step of determining concentrations of a combination of the following five markers: CCL22, GFAP, MDK, NRGN and SPP1 in a sample collected from the said subject at the first time point;
(b) a step of determining the concentrations of the above-mentioned combination of markers in a sample collected from the subject at a second time point;
(c) a step of determining risk factors using the concentrations determined in steps (a) and (b), comparing them with the predetermined cut-off point and, on this basis, determining whether the subject responds to the treatment for glioblastoma multiforme,

wherein the cut-off point is determined by analyzing the concentrations determined in samples collected from healthy subjects, samples collected from subjects suffering from glioblastoma multiforme, and/or samples collected from subjects suffering from another type of tumor.

3.   The method of claim 2, wherein steps (a), (b) and (c) are repeated.

4.   A method of monitoring the progression or recurrence of glioblastoma multiforme in a subject, comprising

(a) a step of determining concentrations of a combination of the following five markers: CCL22, GFAP, MDK, NRGN and SPP1 in a sample collected from the said subject at the first time point;
(b) a step of determining the concentrations of the above-mentioned combination of markers in a sample collected from the subject at a second time point;
(c) a step of determining risk factors using the concentrations determined in steps (a) and (b), comparing them with the predetermined cut-off point and, on this basis, determining the progression or recurrence of glioblastoma multiforme,

wherein the cut-off point is determined by analyzing the concentrations determined in samples collected from healthy subjects, samples collected from subjects suffering from glioblastoma multiforme, and/or samples collected from subjects suffering from another type of tumor.

5.   The method of claim 4, wherein steps (a), (b) and (c) are repeated.

6.   The method of any one of claims 1-5, wherein the sample collected from the subject is whole blood, plasma or serum.

7.   The method of claim 6, wherein the sample is plasma.

8. Use of a combination of markers CCL22, GFAP, MDK, NRGN and SPP1 for the *in vitro* diagnosis of glioblastoma multiforme in a subject.

9. The use of claim 8, wherein diagnosing comprises determining concentrations of the combination of the markers in a sample collected from the subject selected from whole blood, plasma, or serum.

10. Use of a combination of markers CCL22, GFAP, MDK, NRGN and SPP1 to monitor *in vitro* a response of a subject to a treatment of glioblastoma multiforme.

11. The use of claim 10, wherein monitoring comprises determining concentrations of the combination of the markers in a sample collected from the subject selected from whole blood, plasma, or serum.

12. Use of a combination of markers CCL22, GFAP, MDK, NRGN and SPP1 for *in vitro* monitoring the progression or recurrence of glioblastoma multiforme in a subject.

13. The use of claim 12, wherein monitoring comprises determining concentrations of the combination of the markers in a sample collected from the subject selected from whole blood, plasma, or serum.

14. The use of any one of claims 9, 11 or 13, wherein the sample collected from the subject is plasma.

## Fig. 1A

**Fig. 1B**

AUC: 0,538

**Fig. 1C**

**Fig. 1D**

AUC: 0,850, p < 7.131768e-05

**Fig. 2A**

**Fig. 2B**

AUC: 0,940

**Fig. 2C**

**Fig. 2D**

AUC: 0,900, p < 2.469146e$^{-06}$

**Fig. 3A**

**Fig. 3B**

AUC: 0,790

**Fig. 3C**

**Fig. 3D**

AUC: 0.99706, p < 1.073191e-07

## Fig. 4A

**Fig. 4B**

**Fig. 4C**

AUC: 0,740

AUC: 0.740 (0.719-0.761)

**Fig. 3D**

AUC: 0.909

AUC: 0.909 (0.815-1.000)

## Fig. 5A

**Fig. 5B**

**Fig. 5C**

AUC: 0,900

**Fig. 5D**

AUC: 0, 0.9176, p < 1.126475e-06

**Fig. 6A**

AUC: 0.9970588, p < 1.257437e-10

**Fig. 6B**

AUC: 0.9910216, p < 3.504412e-105

**Fig. 7A**
**amygdala**
AUC: 0.9967697
p < 2.064898e-33

**Fig. 7B**
**anterior cingulate cortex**
AUC: 0.9966613
p < 1.182566e-37

**Fig. 7C**
**caudate (basal ganglia)**
AUC: 0.9944733
p < 4.932078e-44

**Fig. 7D**
**cerebellar hemispheres**
AUC: 0.9945589
p < 4.360108e-40

**Fig. 7E**
**cerebellum**
AUC: 0.9928622
p < 5.392478e-46

**Fig. 7F**
**brain cortex**
AUC: 0.9991968
p < 7.100763e-44

**Fig. 7G**
**brain frontal cortex**
AUC: 0.9993659
p < 2.313768e-41

**Fig. 7H**
**hippocampus**
AUC: 0.991179
p< 3.765356e-37

**Fig. 7I**
**hypothalamus**
AUC: 0.9800911
p < 4.794586e-35

**Fig. 7J**
**nucleus accumbens**
**(basal ganglia)**
AUC: 0.9943234
p < 8.029727e-43

GBM vs Brain - Hypothalamus

Nucleus Accumbens (Basal Ganglia)

**Fig. 7K**
**putamen (basal ganglia)**
AUC: 0.9997769
p < 1.621526e-37

**Fig. 7L**
**substantia nigra**
AUC: 0.9367998
p < 4.056418e-23

vs Brain - Putamen (Basal Ganglia)

GBM vs Brain - Substantia Nigra

**Fig. 8A**
**adrenocortical cancer**
AUC: 0.9996088
p < 2.711252e-36

**Fig. 8B**
**bladder urothelial carcinoma**
AUC: 0.9999112
p < 4.765772e-79

**Fig. 8C**
**brain lower grade**
**gliomas**
AUC: 0.8720887
p < 1.114572e-47

**Fig. 8D**
**breast invasive carcinoma**
0.9999671
p < 2.635857e-96

**Fig. 8E**
**cervical & endocervical cancer**
AUC: 0.999626
p < 3.120234e-72

**Fig. 8F**
**cholangiocarcinoma**
AUC: 1
p < 2.832552e-21

**Fig. 8H**
**colon adenocarcinoma**
AUC: 1
p < 5.373056e-71

**Fig. 8I**
**diffuse large B-cell**
**lymphoma**
AUC: 0.9997437
p < 7.211884e-26

**Fig. 8J**
**esophageal carcinoma**
AUC: 1
p < 1.005054e-58

**Fig. 8K**
**head & neck squamous cell carcinoma**
AUC: 0.9999421
p < 3.022455e-84

**Fig. 8L**
**kidney chromophobe cell carcinoma**
AUC: 0.9994524
p < 9.217083e-33

**Fig. 8Ł**
**kidney clear cell carcinoma**
AUC: 0.9997844
p < 1.309361e-84

**Fig. 8M**
**kidney papillary cell carcinoma**
AUC: 0.9990828
p < 1.172602e-70

**Fig. 8N**
**hepatocellular carcinoma**
AUC: 0.9992368
p < 9.780968e-77

**Fig. 8O**
**lung adenocarcinoma**
AUC: 0.999883
p < 4.930538e-84

**Fig. 8P**
**lung squamous cell carcinoma**
AUC: 0.9998548
p < 2.401628e-83

**Fig. 8Q**
**mesothelioma**
AUC: 0.9984767
p < 4.724991e-39

**Fig. 8R**
**neuroblastoma**
AUC: 0.9994762
p < 2.75471e-55

**Fig. 8S**
**ovarian serous**
**cystadenocarcinoma**
AUC: 0.9967128
p < 4.074294e-79

**Fig. 8T**
**pancreatic adenocarcinoma**
AUC: 0.9996635
p < 3.374266e-58

**Fig. 8U**
**pheochromocytoma**
**& paraganglioma**
AUC: 1
p < 1.005054e-58

**Fig. 8V**
**prostate adenocarcinoma**
AUC: 1
p < 2.603226e-83

**Fig. 8W**
**rectum adenocarcinoma**
AUC: 1
p < 6.274361e-41

**Fig. 8X**
**sarcoma**
AUC: 0.9998161
p < 2.32602e-68

**Fig. 8Y**
**skin cutaneous melanoma**
AUC: 1
p < 3.758941e-82

**Fig. 8Z**
**stomach adenocarcinoma**
AUC: 0.9998981
p < 2.005147e-79

**Fig. 8Ą**
**testicular germ cell**
**tumor**
AUC: 0.9968315
p < 1.557031e-53

## Fig. 8Ć
### thymoma
AUC: 0.9998988
p < 2.94092e-47

## Fig. 8Ę
### thyroid carcinoma
AUC: 0.9999765
p < 6.020034e-84

## Fig. 8Ń
### uterine carcinosarcoma
AUC: 0.99461
p < 4.256348e-29

## Fig. 8Ó
### uterine corpus endometrioid carcinoma
AUC: 0.9961393
p < 1.047156e-57

## Fig. 8Ś
### uveal melanoma
AUC: 0.9942047
p < 3.795267e-36

## Fig. 8Ż
### Wilms' tumor
AUC: 0.9995697
p < 1.034614e-48

## Fig. 8Ź
### clear cell sarcoma
### of the kidney
AUC: 1
p < 1.020923e-09

EP 4 538 706 A1

**Tumor resection** ------------------------------------------▶ **palliative care**

histopathological confirmation of GBM

very unfavorable prognostic factors or inability to consent to treatment

then early (<48 hours) postoperative imaging

**progression/relapse**

treatment determined by Kamofsky scale, neurological function and previous treatment:
- repeat surgery
- alkylating chemotherapy
- repeat radiotherapy
- experimental therapy

Occurrence of symptoms
- referral to a doctor
- imaging diagnostics

**Adjuvant treatment**
(6 cycles of chemotherapy)

**FOLLOW-UP**
Neurological and imaging examination at 3-4 month intervals

Initial diagnosis and treatment plan development

**Week 6**   **Week 8**

**Day 0** Radiotherapy cycles with concurrent daily chemotherapy

**Week 30**

**palliative care**

**Collection of material for assessment of biomarker levels**

Material is collected every 30 days, if the result is positive the patient is referred for imaging and treatment

**LUMINEX ANALYSIS**

**LUMINEX ANALYSIS**

monitoring biomarker levels in comparison with the pre-resection state allowing for assessment of treatment efficacy

**LUMINEX ANALYSIS**

**LUMINEX ANALYSIS**

preliminary assessment of GBM biomarker levels in plasma necessary for further monitoring of their levels

monitoring of biomarker levels in comparison with the state before resection and during treatment performed between follow-up visits allowing for monitoring of disease progression/relapse

Biomarker levels do not indicate the presence of GBM

Biomarker levels indicate the presence of GBM

Patient for routine diagnostics

Patient with suspected GBM as a priority for imaging diagnostics

The marker levels change towards the range of the healthy population

Marker levels remain unchanged or increase in GBM patients

Marker levels do not indicate tumor growth

Marker levels **indicate** tumor growth

Positive response to treatment

Negative response to treatment

Maintaining a schedule of follow-up visits

Urgent diagnostic imaging and neurological examination

Fig. 9

EP 4 538 706 A1

# EUROPEAN SEARCH REPORT

**Application Number**

EP 24 20 6734

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2021/222253 A1 (UYTINGCO CEDRIC [US]) 22 July 2021 (2021-07-22) | 1-5,8, 10,12 | INV. G01N33/574 |
| A | * Table 3, 5, 8 * | 6,7,9, 11,13,14 | |
| Y | MI ZHOU ET AL: "Serum macrophage-derived chemokine/CCL22 levels are associated with glioma risk, CD4 T cell lymphopenia and survival time", INTERNATIONAL JOURNAL OF CANCER, JOHN WILEY & SONS, INC, US, vol. 137, no. 4, 2 February 2015 (2015-02-02), pages 826-836, XP071289267, ISSN: 0020-7136, DOI: 10.1002/IJC.29441 | 1-5,8, 10,12 | |
| A | * abstract ; Table 1-3 ; Fig 1 * | 6,7,9, 11,13,14 | |
| Y | US 2021/140982 A1 (UYTINGCO CEDRIC [US]) 13 May 2021 (2021-05-13) | 1-5,8, 10,12 | |
| A | * Table 15-17 * | 6,7,9, 11,13,14 | TECHNICAL FIELDS SEARCHED (IPC) |
| A | P. SREEKANTHREDDY ET AL: "Identification of Potential Serum Biomarkers of Glioblastoma: Serum Osteopontin Levels Correlate with Poor Prognosis", CANCER EPIDEMIOLOGY, BIOMARKERS & PREVENTION, vol. 19, no. 6, 1 June 2010 (2010-06-01), pages 1409-1422, XP055306873, ISSN: 1055-9965, DOI: 10.1158/1055-9965.EPI-09-1077 * abstract ; pg 1413, col 2, para bridging pg 1414 ; Fig 6 * | 6,7,9, 11,13,14 | G01N |

The present search report has been drawn up for all claims

| Place of search | Date of completion of the search | Examiner |
|---|---|---|
| The Hague | 24 February 2025 | Vadot-Van Geldre, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 20 6734

This annex lists the patent family members relating to the patent documents cited in the above-mentioned European search report.
The members are as contained in the European Patent Office EDP file on
The European Patent Office is in no way liable for these particulars which are merely given for the purpose of information.

24-02-2025

| Patent document cited in search report | Publication date | Patent family member(s) | Publication date |
|---|---|---|---|
| US 2021222253 A1 | 22-07-2021 | NONE | |
| US 2021140982 A1 | 13-05-2021 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20140045915 A1
- US 20220244263 A1

**Non-patent literature cited in the description**

- **VITOVCOVA, B. et al.** Biology of Glioblastoma Multiforme-Exploration of Mitotic Catastrophe as a Potential Treatment Modality. *Int J Mol Sci*, 2020, vol. 21 (15)
- **PEARSON, J.R.D.** ; **T. REGAD**. Targeting cellular pathways in glioblastoma multiforme. *Signal Transduct Target Ther*, 2017, vol. 2, 17040
- **CHANG, S.M. et al.** Patterns of care for adults with newly diagnosed malignant glioma. *JAMA*, 2005, vol. 293 (5), 557-64
- **JONES, D.R.** Measuring midkine: the utility of midkine as a biomarker in cancer and other diseases. *Br J Pharmacol*, 2014, vol. 171 (12), 2925-39
- **LANGE, R.P. et al.** Evaluation of eight plasma proteins as candidate blood biomarkers for malignant gliomas. *Cancer Invest*, 2014, vol. 32 (8), 423-9
- **YOKOTA, T. et al.** Identification of histological markers for malignant glioma by genome-wide expression analysis: dynein, alpha -PIX and sorcin. *Acta Neuropathol*, 2006, vol. 111 (1), 29-38
- Circulating Midkine in Malignant, Inflammatory, and Infectious Diseases: A Systematic Review. **KRZYSTEK-KORPACKA, M.** ; **M. MATUSIEWICZ**. Midkine: From Embryogenesis to Pathogenesis and Therapy. Springer, 2012, 69-85
- **CHENG, Y.P. et al.** Midkine high-grade expression gliomas: Correlation of this novel marker with proliferation and survival in human gliomas. *Surg Neurol Int*, 2014, vol. 5, 78
- **MIDDELDORP, J.** ; **E.M. HOL**. GFAP in health and disease. *Threshold Neurobiol*, 2011, vol. 93 (3), 421-43
- **PLOG, B.A. et al.** Biomarkers of traumatic injury are transported from brain to blood via the glymphatic system. *J Neurosci*, 2015, vol. 35 (2), 518-26
- **ABDELHAK, A. et al.** Blood GFAP as an emerging biomarker in brain and spinal cord disorders. *Nat Rev Neurol*, 2022, vol. 18 (3), 158-172
- **TICHY, J. et al.** Prospective evaluation of serum glial fibrillar acidic protein (GFAP) as a diagnostic marker for glioblastoma. *J Neurooncol*, 2016, vol. 126 (2), 361-9
- **NICHOLS, N.R. et al.** GFAP mRNA increases with age in rat and human brain. *Neurobiol Aging*, 1993, vol. 14 (5), 421-9
- **XIANG, Y. et al.** Neurogranin: A Potential Biomarker of Neurological and Mental Diseases. *Front Aging Neurosci*, 2020, vol. 12, 584743
- **DE VOS, A. et al.** C-terminal neurogranin is increased in cerebrospinal fluid but unchanged in plasma in Alzheimer's disease. *Alzheimers Dement*, 2015, vol. 11 (12), 1461-1469
- **KVARTSBERG, H. et al.** Characterization of the postsynaptic protein neurogranin in paired cerebrospinal fluid and plasma samples from Alzheimer's disease patients and healthy controls. *Alzheimers Res Ther*, 2015, vol. 7 (1), 40
- **KUSDOGAN, M. et al.** The diagnostic and prognostic value of serum neurogranin in acute ischemic stroke. *J Stroke Cerebrovasc Dis*, 2023, vol. 32 (2), 106889
- **LANGE, R.P. et al.** Evaluation of eight plasma proteins as candidate blood based biomarkers for malignant gliomas. *Cancer Invest*, 2014, vol. 32 (8), 423-9
- **ANZ, D. et al.** Suppression of intratumoral CCL22 by type and interferon inhibitions migration of regulatory T cells and blocks cancer progression.. *Cancer Res*, 2015, vol. 75 (21), 4483-93
- **RAPP, M. et al.** CCL22 controls immunity by promoting regulatory T cell communication with dendritic cells in lymph nodes.. *J Exp Med*, 2019, vol. 216 (5), 1170-1181
- **ROHRLE, N.** ; **M.M.L. KNOTT** ; **D. ANZ**. CCL22 Signaling in the Tumor Environment. *Adv Exp Med Biol*, 2020, vol. 1231, 79-96
- **WIEDEMANN, GM et al.** Cancer cell-derived IL-1alpha induces CCL22 and the recruitment of regulatory T cells. *Oncoimmunology*, 2016, vol. 5 (9), e1175794

- **ZHOU, M. et al.** Serum macrophage-derived chemokine/CCL22 levels are associated with glioma risk, CD4 T cell lymphopenia and survival time. *Int J Cancer*, 2015, vol. 137 (4), 826-36
- **VAN DIJCK, A. et al.** Reduced serum levels of proinflammatory chemokines in fragile X syndrome. *BMC Neurol*, 2020, vol. 20 (1), 138
- **SKRZYPKOWSKA, M. et al.** Cytokines and chemokines multiplex analysis in patients with low disease activity rheumatoid arthritis. *Rheumatol Int*, 2022, vol. 42 (4), 609-619
- **KAHLES, F.** ; **H.M. FINDEISEN** ; **D. BRUEMMER**. Osteopontin: A novel regulator at the cross roads of inflammation, obesity and diabetes. *Molecular metabolism*, 2014, vol. 3 (4), 384-393
- **KARIYA, Y.** ; **Y. KARIYA**. Osteopontin in Cancer: Mechanisms and Therapeutic Targets. *International Journal of Translational Medicine*, 2022, vol. 2 (3), 419-447
- **SREEKANTHREDDY, P. et al.** Identification of potential serum biomarkers of glioblastoma: serum osteopontin levels correlate with poor prognosis. *Cancer Epidemiol Biomarkers Prev*, 2010, vol. 19 (6), 1409-22
- **BLASBERG, JD et al.** Reduction of elevated plasma osteopontin levels with resection of non-small-cell lung cancer. *J Clin Oncol*, 2010, vol. 28 (6), 936-41
- **HAO, C. et al.** Human osteopontin: Potential clinical applications in cancer (Review). *Int J Mol Med*, 2017, vol. 39 (6), 1327-1337
- **ANBORGH, PH et al.** Role of plasma osteopontin as a biomarker in locally advanced breast cancer. *Am J Transl Res*, 2015, vol. 7 (4), 723-32
- **KOOPMANN, J. et al.** Evaluation of osteopontin as a biomarker for pancreatic adenocarcinoma.. *Cancer Epidemiol Biomarkers Prev*, 2004, vol. 13 (3), 487-91
- **KISS, T. et al.** The role of osteopontin expression in melanoma progression. *Tumour Biol*, 2015, vol. 36 (10), 7841-7